# EUROPEAN PATENT SPECIFICATION

(11) **EP 2 051 987 B1**
(45) Date of publication and mention of the grant of the patent: **19.11.2014**
(21) Application number: 07811339.6
(22) Date of filing: 16.08.2007
(51) Int. Cl.: A61K 38/17, A61K 31/5377, A61K 31/436, A61K 45/06, A61P 37/06

(54) **USE OF CD83 IN COMBINATION THERAPIES**
VERWENDUNG VON CB83 IN KOMBINATIONSTHERAPIEN
UTILISATION DE CD83 DANS DES POLYTHÉRAPIES

(30) Priority: 18.08.2006 US 838812 P; 04.05.2007 US 927377 P
(43) Date of publication of application: 29.04.2009
(60) Divisional application: 14167252.7
(73) Proprietor: Argos Therapeutics, Inc., Durham, NC 27704 (US)
(72) Inventor: NICOLETTE, Charles, Durham, North Carolina 27703 (US); BRAND, Stephen, Chapel Hill, North Carolina 27516 (US); ZHONG, Zhen, London, Ontario N6A 5N5 (CA); WANG, Hao, London, Ontario N5X 4H2 (CA); ARP, Jacqueline, London, Ontario N6K 2T1 (CA); RAMCHARRAN, Siobhan, Mississauga, Ontario L4T 1T8 (CA); BAROJA, Mirin Lorea, London, Ontario N6G 5H3 (CA)
(74) Representative: Taylor, Kate Laura
(86) International application number: PCT/US2007/018048
(87) International publication number: WO 2008/024242

(56) References cited:
- WO-A1-96/32965
- WO-A1-97/29781
- WO-A1-2004/016284
- WO-A2-2004/046182
- US-A1- 2003 219 436
- US-A1- 2004 110 673
- US-B2- 7 169 898
- LECHMANN M ET AL: "Role of CD83 in the immunomodulation of dendritic cells", INTERNATIONAL ARCHIVES OF ALLERGY AND IMMUNOLOGY, KARGER AG, CH, vol. 129, no. 2, 20 October 2002 (2002-10-20), pages 113-118, XP009015843, ISSN: 1018-2438, DOI: DOI:10.1159/000065883
- BESSMERTNY ET AL: "94 Tacrolimus (FK-506) and mycophenolate mofetil (MMF) GVHD prophylaxis in allogeneic SCT (alloSCT) recipients: Altered MMF pharmacokinetics (PK) associated with AGVHD", BIOLOGY OF BLOOD AND MARROW TRANSPLANTATION, KLUGE CARDEN JENNINGS PUBLISHING, CHARLOTTESVILLE, VA, US, vol. 9, no. 2, 1 February 2003 (2003-02-01), page 93, XP005433264, ISSN: 1083-8791, DOI: DOI:10.1016/S1083-8791(03)80095-2
- SITTENGER M.: 'Tissue engineering and autologous transplant formation: practical approaches with resorbable biomaterials and new cell culture techniques' BIOMATERIALS vol. 17, 1996, pages 237 - 242, XP004032789
- KREIS H.: 'Sirolimus in association with mycophenolate mofetil induction for the prevention of acute graft rejection in renal allograft recipients' TRANSPLANT vol. 69, no. 7, April 2000, pages 1252 - 1260, XP008103394
- NEUMANN I.: 'Pharmacokinetics of Mycophenolate Mofetil in Patients with Autoimmune Disease Compared Renal Transplant Recipients' vol. 14, 2003, XP008100909

## Description

### FIELD OF THE INVENTION

The present invention relates to improved methods of treating a subject so as to suppress or prevent undesired immune responses. The methods comprise the use of CD83 and tolerize a subject to a therapeutic composition. For example, the methods can be used to prevent rejection of transplanted tissue.

### BACKGROUND OF THE INVENTION

The immune system of mammals is capable of mounting a protective response to a very large number of foreign antigens. Unfortunately, this very responsiveness can cause difficulties when it is directed to compounds that are intended for therapeutic use-including, for example, transplanted tissue, gene therapy vectors, and compounds (particularly proteins) used for therapy of certain disorders and diseases. For example, patients afflicted with hemophilia B can develop an immune response to the therapeutic Factor IX as well as to a gene transfer vector used to express Factor IX in these patients (see, *e.g*., Dobryzynski et al. (2006) Proc. Nat'l. Acad Sci. USA 103: 4592-4597). Similarly, patients treated with therapeutic antibodies (e.g., Rituxan^{®} and Bexxar^{®}) can develop an immune response against those therapeutic antibodies which renders them ineffective and poses an additional risk to the patient.

Unfortunately, present treatments to prevent or eliminate an undesirable immune response typically involve the use of compounds that cause a global suppression of the immune system (see, e.g., Chapter 14 of Janeway et al., eds. (2001) Immunobiology (5th ed., Garland Publishing, New York, NY)). These treatments put the patient at risk for future infections (see, *e.g*., Gottschalk et al. (2005) Annu. Rev. Med. 56: 29-44) and generally have undesirable and serious side effects. Some studies that combined different treatments showed that some combinations actually exacerbate toxicity (see, *e.g.,* Andoh et al. (1996) Transplantation 62: 311-316). Presently available treatments can also require treatment for a prolonged period of time--in some cases (*e.g*., tissue transplantation), for the remainder of the patient's life.

There are also many other instances in which a focused and time-limited suppression of the immune system is desirable, for example in treating allergic reactions and hypersensitivity reactions (see, *e.g.,* Chapter 12 of Janeway et al., eds. (2001) Immunobiology (5th ed., Garland Publishing, New York, NY)), such as contact dermatitis, chronic asthma, drug allergies, and systemic anaphylaxis. Generally, clinical treatment to suppress these responses also has a broad effect (see Janeway *et al., id*.). Another example is seen with B-cell-mediated responses. B cells are the primary mediators of antibody-mediated transplant rejection and also serve as antigen presenting cells. However, current therapy to prevent adverse effects of an unwanted B-cell-mediated response is limited to depletion using monoclonal antibodies (mAb) against CD20, and there exists no effective therapy to prevent B cell activation and/or differentiation. Other currently-used treatments for undesirable immune responses use specific inhibitors to block the synthesis or effects of inflammatory mediators produced by mast cells. Some treatments designed to induce desensitization involve the injection of specific antigen(s); these treatments are believed to divert the immune response to the allergen from a T_{H}2 to a T_{H}1 type response so that IgG is produced in place of IgE. These treatments have succeeded in many cases, but not in all.

Many forms of immunity are mediated by T cells, including cell lympholysis, delayed type hypersensitivity (DTH), transplantation rejection, and allograft rejection (see, *e.g*., Paul (1993) Fundamental Immunology (Raven Press, New York, New York) (3d ed.)). T cell behavior is affected by dendritic cells ("DCs"), which are the professional antigen-presenting cells of the immune system and can dramatically stimulate a T cell response (sometimes also referred to herein as "immunostimulatory dendritic cells"; see, *e.g*., Banchereau and Schmitt, eds. (1995) Dendritic Cells in Fundamental and Clinical Immunology, Vol. 2 (Plenum Press, New York); Schuler et al. (2003) Curr. Opin. Immunol. 15: 138-147; U.S. Pat. App. No. 10/287,813).

Under some circumstances, dendritic cells also play an important role in immune tolerance. In this context, they are sometimes referred to as "tolerogenic dendritic cells" (see, *e.g*., Steinman et al. (2003) Ann. Rev. Immunol. 21: 685-711; Kubach et al. (2005) Int. J. Hematol. 81: 197-203). Unfortunately, dendritic cells are rare in peripheral blood, so certain applications may necessitate an *ex vivo* method for producing therapeutic quantities of tolerogenic dendritic cells. Methods for making immunostimulatory DCs have been described (see, *e.g.,* U.S. Pat. No. 6,274,378), but these DCs are not tolerogenic. Other types of cells have also been shown to have a role in tolerance, including "regulatory T cells" (see, *e.g*., Boehmer (2005) Nat. Immunol. 6: 338-344). Studies in rodents have demonstrated a unique CD4⁺ CD25⁺ population of professional regulatory (or "suppressor") T cells that actively and dominantly prevent both the activation and the effector function of autoreactive T cells (Sakaguchi et al. (1995) J. Immunol. 155: 1151-1164; Takahashi et al. (1998) Int. Immunol. 10: 1969-1980; Itoh et al. (1999) J. Immunol. 162: 5317-5326). The elimination or inactivation of these regulatory T cells has been shown to result in severe autoimmune disease and to enhance immune responses to alloantigens and tumors (see also, Shimizu et al. (1999) J. Immunol. 163: 5211-5218). Recent work has identified regulatory T cells in humans (U.S. Pat. App. No. 10/661,804). The preparation of human T cells with regulatory properties but which are not CD4⁺ CD25⁺ T cells has also been reported (see U.S. Pat. App. No. 10/618,134).

CD83 is a molecule from the immunoglobulin ("Ig") superfamily of proteins (see, *e.g*., Zhou et al. (1999) J. Immunol. 149: 735-742; see also U.S. Pat. No. 7,169,898; for review, see Fujimoto and Tedder ((2006) J. Med. Dent. Sci. 53: 86-91). While the precise function of CD83 remains to be determined, a soluble form of CD83 has been reported to bind to both immature and mature dendritic cells (see Lechmann et al. (2001) (J. Exp. Med. 194: 1813-1821). The authors also reported that this protein decreased the expression of CD80 and CD83 by dendritic cells matured *in vitro* and that it inhibited the ability of dendritic cells to stimulate T cells *in vitro* (see also, Lechmann et al. (2002) Trends in Immunology 23: 273-275). Others have reported the presence of a soluble form of CD83 *in vivo* (see, *e.g*., Hock et al. (2002) Int. Immunol. 13: 959-967). Studies with HSV-1-infected DCs demonstrate that viral infection leads to the degradation of CD83 and inhibition of CD83 mRNA transport; this possible mechanism of escape by HSV-1 further supports the importance of CD83 to DC biology (see, *e.g*., Kruse et al. (2000) J. Virol. 74: 7127-7136). Kruse et al. (2000) (J. Exp. Med. 191: 1581-1589) reported that GC7 (N(1)-guanyl-1,7-diaminoheptane) interfered with the nucleocytoplasmic translocation of CD83 mRNA, preventing the surface expression of CD83 and significantly inhibiting the activation of T lymphocytes by these DCs.

CD83 is a single-chain glycoprotein of about 45 kDa, and the immature form of CD83 includes a signal peptide which is removed on insertion of the protein into the membrane. Mature CD83 includes three structural domains: an extracellular domain; a transmembrane domain; and a cytoplasmic domain (see, *e.g.,* WO 2004/046182, which correctly identifies the extracellular domain). The CD83 extracellular domain comprises a single Ig-like (V-type) domain which is encoded by at least two exons (see, *e.g*., Zhou et al. (1999) J. Immunol. 149: 735-742; GenBank ID #Z11697) and is expressed very strongly on the cell surface of native mature dendritic cells ("mDCs").

U.S. Pat. No. 5,316,920 and the corresponding WO 93/21318 describe a CD83 protein (referred to as "HB15") and "cDNA sequences encoding the HB15 protein or portions thereof, including any of its specific domains, ligand binding fragments or immunospecific fragments" (col. 2, lines 23-25). The patent also discusses the use of "antibodies reactive with HB 15 and methods of using anti-HB15 antibodies, or other antagonists to HB 15 function, to treat an immunological disorder, disease or syndrome" (see Abstract). The patent further speculates (col. 3, lines 6-12) that "[t]he HB15 protein, immunospecific or ligand binding fragments or specific domains thereof, or other antagonists to HB15 that interfere with HB 15 function, can be used therapeutically to modify or inhibit the development or progression of an immune response or cellular interaction, or to deliver drugs, toxins, or imaging agents to cells that express HB 15."

U.S. Pat. No. 5,710,262 (a continuation-in-part of the application that was granted as U.S. Pat. No. 5,316,920) and the corresponding WO 95/29236 are directed to CD83 proteins and discuss the comparison between human and mouse proteins (col. 2, lines 46-48). These patents and application further are directed to "methods of producing human HB15 or a mammalian homolog of HB15" (col. 3, lines 5-6), the production of antibodies (col. 4, lines 35-37), and the use of an HB 15 antibody to "deliver drugs, toxins, or imaging agents to cells that express HB 15" (col. 4, lines 38-39).

WO 97/29781 is directed to "a method of stimulating a humoral immune response, comprising administering a CD83 reagent and an antigen, in a pharmaceutically acceptable carrier, wherein the CD83 stimulates production of antigen-specific antibodies" (p. 1). The application states that "[i]n a preferred embodiment, CD83 reagents are DNA's that encode, and CD83 peptides that comprise, the extracellular domain of CD83" (p. 2). The application also discusses the use of CD83 antibodies to inhibit undesirable antigen specific responses in a ' mammal and states that "[s]uch methods...are useful in preventing or treating autoimmune disease as well as tissue or organ transplant rejection, and in treatment or prevention of allergy or asthma" (p. 3).

WO 2004/046182 and the corresponding U.S. Patent No. 7,169,898 (both of which are hereby incorporated in their entirety by reference) correctly identify the extracellular domain of CD83 and suggest "the use of a soluble form of a member of the CD83 family of proteins...for the treatment or prevention of a disease or medical condition caused by the dysfunction or undesired function of a cellular immune response involving dendritic cells, T cells, and/or B cells" (WO 2004/046182, p. 7). The PCT application states (p. 12) that a form of CD83 induced an altered surface marker expression pattern in both immature and mature dendritic cells. Further, "soluble forms of the members of the CD83 family of proteins of the present invention are capable of disrupting the interaction of dendritic cells to T cells and/or B cells and/or inhibiting the formation of dendritic cell-T cell clusters." (p. 14; see also Sénéchal et al. (2004) Blood 103: 4207-4215). The application further discloses that mice treated with hCD83ext did not develop the typical paralysis associated with experimental autoimmune encephalitis ("EAE") (p. 12; see also Zinser et al. (2004) J. Exp. Med. 200: 345-351).

In view of the above, Applicants recognized a need for safe and effective treatments that can suppress an immune response to a particular composition without producing global suppression of the immune response and without causing other harm to the patient.

### SUMMARY OF THE INVENTION

In one aspect the invention provides soluble CD83 for use to prevent, cure, or alleviate at least one symptom of a disease or disorder caused by the dysfunction or undesired function of an immune response in combination with a first immunosuppressive compound, wherein said first immunosuppressive compound is selected from cyclosporine, tacrolimus, sirolimus, and anti-CD45RB mAb, wherein said disease or disorder is selected from the group consisting of an allergy; asthma; rejection of transplanted tissue; an immune response to a chronically administered substance; an autoimmune disease; and AIDS.

In a further aspect the invention provides a combination of soluble CD83 and a first immunosuppressive compound for use to prevent, cure, or alleviate at least one symptom of a disease or disorder caused by the dysfunction or undesired function of an immune response, wherein said first immunosuppressive compound is selected from the group consisting of cyclosporine, tacrolimus, sirolimus, and anti-CD45RB mAb, wherein said disease or disorder is selected from the group consisting of an allergy; asthma; rejection of transplanted tissue; an immune response to a chronically administered substance; an autoimmune disease; and AIDS.

In a further aspect the invention provides use of a combination of soluble CD83 and a first immunosuppressive compound for treating a tissue to be transplanted after removal from a donor and prior to transplantation, wherein said first immunosuppressive compound is selected from the group consisting of cyclosporine, tacrolimus, sirolimus, and anti-CD45RB mAb.

### BRIEF DESCRIPTION OF THE DRAWINGS

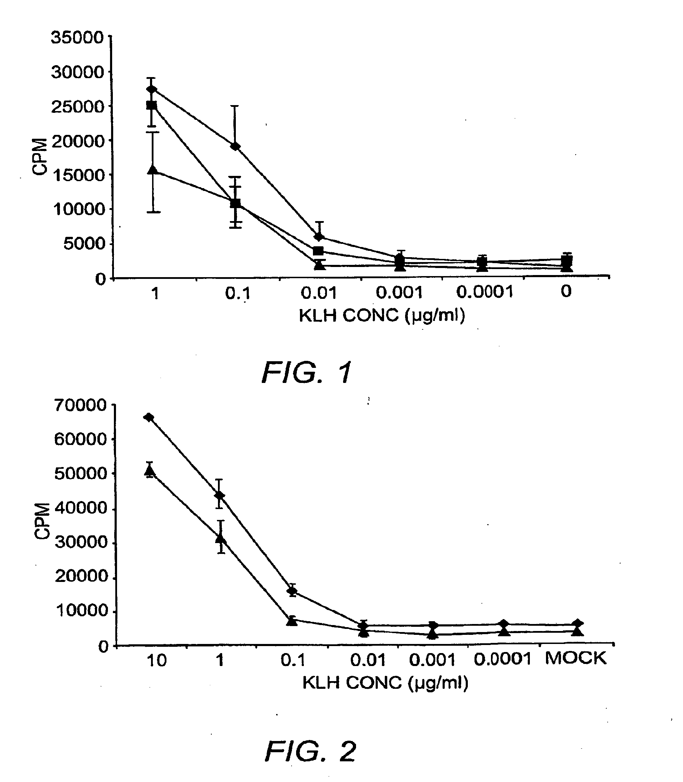

Figure 1 (see Example 2) shows [³H]-thymidine uptake in a T-cell proliferation assay as a function of the concentration of keyhole limpet hemocyanin (KLH) that was administered to the mice from which the cells were derived. Briefly, animals were treated with sCD83 and then injected with KLH; after 28 days, spleen cells were removed and restimulated with different doses of KLH. Incorporation of ³H-thymidine is shown as a function of KLH concentration (µg/ml) for cells obtained from animals that were treated with sCD83 (diamonds) as well as for control animals that were untreated (squares) or treated with BSA (triangles).
Figure 2 shows [³H]-thymidine uptake in a T-cell proliferation assay as a function of the concentration of keyhole limpet hemocyanin (KLH) that was administered to the mice from which the cells were derived (see Example 3). Briefly, animals were treated with sCD83 and then injected with KLH; after 10 days, spleen cells were then removed and restimulated with different doses ofKLH. Incorporation of ³H-thymidine is shown as a function of KLH concentration (µg/ml) for cells obtained from animals that were treated with sCD83 (diamonds) as well as for control animals that were mock-inoculated (triangles).

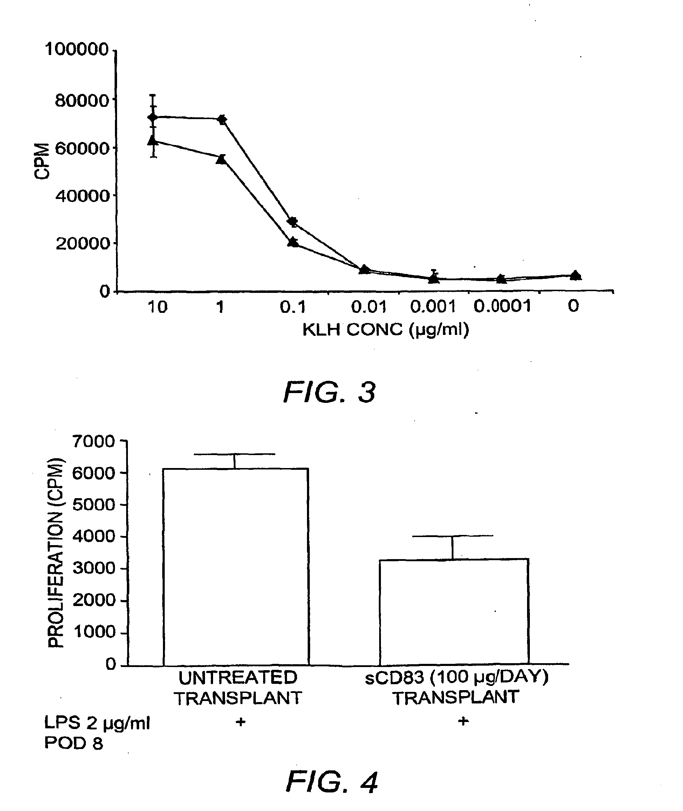

Figure 3 shows [³H]-thymidine uptake in a T-cell proliferation assay as a function of the concentration of keyhole limpet hemocyanin (KLH) that was administered to the mice from which the cells were derived (see Example 3). Briefly, animals were treated with sCD83 and then injected with KLH; after 22 days, spleen cells were then removed and restimulated with different doses ofKLH. Incorporation of ³H-thymidine is shown as a function of KLH concentration (µg/ml) for cells obtained from animals that were treated with sCD83 (diamonds) as well as for control animals that were mock-inoculated (triangles).
Figure 4 shows results of an experiment described in Example 5 and demonstrates that soluble CD83 ("sCD83") suppresses *ex vivo* B-cell proliferation.
Figure 5 shows results of an experiment described in Example 5 and demonstrates that sCD83 suppresses *ex vivo* antibody production by B cells of the transplant recipient. Asterisks indicate p < 0.01.
Figure 6 shows results of an experiment described in Example 7 and demonstrates that sCD83 can prevent the induction of an allo-antibody response by B cells to foreign antigens.
Figure 7 shows results of an experiment described in Example 8 and demonstrates that coadministration of sCD83 with sirolimus ("Rapa") and anti-CD45RB mAb provided long-term survival of transplanted heart tissue.
Figure 8 shows results of an experiment described in Example 8 and demonstrates that coadministration of sCD83 with sirolimus ("Rapa") and anti-CD45RB mAb inhibited DC maturation in transplant recipients. Asterisks indicate p < 0.05.
Figure 9 shows results of an experiment described in Example 8 and, like Figure 9, demonstrates that coadministration of sCD83 with sirolimus ("Rapa") and anti-CD45RB mAb inhibited DC maturation in transplant recipients. Asterisks indicate p < 0.01.
Figure 10 shows results of an experiment described in Example 8 and demonstrates that coadministration of sCD83 with sirolimus ("Rapa") and anti-CD45RB mAb generated regulatory T cells in transplant recipients. Asterisk indicates p < 0.01.
Figure 11 shows results of an experiment described in Example 9 and demonstrates that sCD83 suppressed *in vitro* TNF-α production and surface expression of mDC activation markers by mDCs from cynomolgus monkeys.
Figure 12 shows results of an experiment described in Example 9 and demonstrates that sCD83 moderately suppressed *in vitro* TNF-α production and surface expression of monocyte activation markers.
Figure 13 shows results of an experiment described in Example 9 and indicates that sCD83 promoted the generation of regulatory T cells. The vertical axis shows the percentage of CD4⁺ CD25⁺ iFoxp3⁺ cells.
Figure 14 shows results of an experiment described in Example 9 and indicates that increased numbers of regulatory T cells were generated by dendritic cells treated with soluble CD83.

### DETAILED DESCRIPTION OF THE INVENTION

The inventio discloses methods of using CD83 to treat a subject. Methods described herein include the use of CD83 in combination with at least one other immunosuppressive compound. Disclosed are methods using CD83 in combination with at least one other immunosuppressive compound; also disclosed are compositions comprising CD83 and at least one other immunosuppressive compound. Methods include methods using CD83 with at least one, two, three, four, or five or more immunosuppressive compounds.

In some embodiments, methods disclosed in the invention also comprise the use of other compounds. For example, in some embodiments, at least one therapeutic composition is coadministered to a subject with CD83 to tolerize the subject to the therapeutic composition(s). In some embodiments, methods disclosed in the invention comprise tolerizing a subject by coadministering CD83 and at least one other immunosuppressive compound to the subject along with at least one therapeutic composition. The methods can be used, for example, to induce tolerance to the at least one therapeutic composition or to prevent or reduce the severity of autoimmune diseases. The therapeutic composition may be transplanted tissue. Thus, in some embodiments, the methods disclosed in the invention are used to prevent rejection of transplanted tissue.

The invention also describes methods of tolerizing a subject that comprise producing tolerogenic dendritic cells via maturation in the presence of CD83 and at least one other immunosuppressive compound either *in vivo* or *in vitro (e.g., ex vivo).* The tolerogenic dendritic cells are used to produce regulatory T cells. The tolerogenic dendritic cells and regulatory T cells can be produced *in vivo* or ex *vivo;* cells produced *ex vivo* can then be administered to a subject. The tolerogenic dendritic cells and regulatory T cells can be autologous or can be derived from stem cells. The compositions and methods may be used for tolerization to antigens as well as for treatment or prevention of a disease or medical condition (*e.g.,* a disorder) caused by the dysfunction or undesired function of an immune response, wherein an effective amount of CD83 and at least one other immunosuppressive compound is administered to a subject.

The invention describes complete tolerization of a subject to a therapeutic composition. In some embodiments, the objects of the invention include the prevention, cure, reduction, and/or alleviation of at least one symptom of a disease or disorder caused by the dysfunction or undesired function of an immune response. A subject is considered to be tolerized and/or to have been immunosuppressed (*i.e*., immune tolerance is considered to have been induced or acquired) if at least one of these objects is achieved. Thus, "tolerizing" a subject or inducing "immunosuppression" as used herein means that at least one symptom of a disease or disorder caused by the dysfunction or undesired function of an immune response involving dendritic cells, B cells, or T cells is prevented, cured, reduced, or alleviated in comparison to an untreated control or other appropriate control *(e.g.,* in comparison to the symptom prior to treatment or to the expected severity of the symptom without treatment, if the treatment is intended to prevent the development of or reduce the severity of an immune response). Those of skill in the art are familiar with the selection and application of methods of measurement and evaluation of symptoms as well as with the selection of appropriate controls.

Thus, a subject is considered to be tolerized and/or immunosuppression is considered to have occurred where at least one symptom of a disease or disorder caused by the dysfunction or undesired function of an immune response is reduced or alleviated by at least 10%, 20%, 30%, 40%, 50%, 60%, 70%, 80%, 90%, 95%, or 100% in comparison to an appropriate control. In embodiments where the treatment is intended to reduce the risk of a subject for developing an autoimmune disorder, that risk is reduced or alleviated by at least 10%, 20%, 30%, 40%, 50%, 60%, 70%, 80%, 90%, 95%, or 100% in comparison to an appropriate control; this assessment may be performed statistically on a population of subjects. Where the treatment is intended to tolerize a subject to a therapeutic composition, an undesired function of an immune response is reduced by at least 10%, 20%, 30%, 40%, 50%, 60%, 70%, 80%, 90%, 95%, or 100% in comparison to an appropriate control.

The methods disclosed in the invention are useful for therapeutic purposes and thus are intended to prevent, cure, or alleviate at least one symptom of a disease or disorder caused by the dysfunction or undesired function of an immune response. A symptom of a disease or disorder is considered to be reduced or alleviated if the symptom is decreased, increased, or improved, as appropriate, by at least 10%, 20%, 30%, 40%, 50%, 70%, 90% or more in comparison to an appropriate control, such as in comparison to the symptom prior to treatment or in comparison to the expected severity of the symptom, where the treatment is intended to be preventive. One of skill is familiar with techniques and criteria for evaluating changes in symptoms. Symptoms of diseases or disorders caused by the dysfunction or undesired function of an immune response are known to those in the art and include the following: abnormal histology of a transplanted tissue; abnormal function of a transplanted tissue; brief length of survival time following an event such as, for example, diagnosis or transplantation; abnormally or undesirably high or low level or number of indicator protein(s) or other compound(s) in the blood, such as undesired antibodies or undesired cells (*e.g*., antigen-specific dendritic cells or T cells); abnormally or undesirably high or low level or number of indicator cells in the blood or elsewhere in the body, *e.g*., an undesirably low level or number of regulatory T cells, so that an undesired immune response is initiated or maintained.

Where appropriate, *in vivo* tolerization or tolerance and/or immunosuppression may be measured using *in vitro* assays, such as, for example, in a mixed lymphocyte reaction using cells isolated from a subject. Similarly, tolerization or tolerance and/or immunosuppression achieved in cells *ex vivo* may also be measured in *ex vivo* assays using various types of cells, such as, for example, dendritic cells, T cells, or B cells. If tolerization or tolerance and/or immunosuppression is measured using an *ex vivo* method, tolerization or tolerance is considered to have occurred if the response of the cells to an immune stimulus is decreased by at least 10%, 20%, 30%, 40%, 50%, 70%, 90% or more in comparison to an appropriate control. Suitable assays directly or indirectly measure immune response and are known in the art; they include, but are not limited to: mixed lymphocyte reaction assays; cytotoxicity assays; antibody titer assays; assays for the production of IL-10; assays for the production of TGF-β; evaluation of cell surface markers; and assays for the expression of Foxp3.

The term "soluble CD83" or "sCD83" as used herein refers to a proteinaceous molecule that comprises at least a portion of the extracellular domain of a member of the CD83 family of proteins. In some embodiments, a soluble CD83 protein does not have an amino acid sequence that is capable of anchoring said molecule to the membrane of a cell in which it is expressed. For example, a soluble CD83 protein may include additional residues of the full-length, native CD83 protein which are outside the extracellular domain. The CD83 protein, nucleic acid sequence, and structure are known in the art. The nucleic acid sequence of human CD83 (as set forth in GenBank Accession No. Z11697) is set forth in SEQ ID NO:1. This sequence includes a coding sequence from position 11 to 628 (including the stop codon) and a signal peptide from position 11 to 67. The sequence encoding the mature CD83 peptide extends from position 68 to 625. Restriction enzyme recognition sites are present at the beginning and end of the sequence (at positions 1 to 6 and 1755 to 1760). The amino acid sequence of human CD83 (as set forth in GenBank Accession No. Q01151 and encoded by the nucleic acid sequence set forth in SEQ ID NO:1) is set forth in SEQ ID NO:2.

The extracellular domain of CD83 has been described as including amino acids 20 to 144 of the native, full-length protein. The full-length amino acid sequence of CD83 is set forth in SEQ ID NO:2, and amino acids 20 to 144 of the full-length CD83 are also set forth in SEQ ID NO:4. The portion of the CD83 nucleic acid sequence which encodes the extracellular domain is set forth in SEQ ID NO:3. See also, *e.g*., Zhou et al. (1992) J. Immunol. 149: 735-742; GenBank Accession Number Z 11697; and SwissProt Acc. No. Q01151; WO 2004/046182, herein incorporated by reference in its entirety.

A CD83 protein is "membrane bound"; that is, it comprises at least a portion of the extracellular domain of a member of the CD83 family of proteins and also comprises a protein or peptide having an amino acid sequence that is capable of anchoring it to a membrane, such as the membrane of a cell. As used herein, the term "CD83" encompasses soluble CD83 and membrane-bound CD83.

A CD83 protein will have at least 65%, 70%, 75%, 80%, 85%, 90%, 95%, or more sequence identity to the corresponding portion of the sequence of the human CD83 protein set forth in SEQ ID NO:2) and/or SwissProt Acc. No. Q01151. Thus, the term "CD83" encompasses the murine CD83 protein (also called "HB 15"; see, *e.g*., Berchtold et al. (1999) FEBS Lett. 461: 211-216). A CD83 protein comprises a fragment of at least 15, 20, 25, 30, 35, 40, 45, 50, 55, 60, 75, 100, or more consecutive amino acids from the native, full-length CD83 protein, or comprises an amino acid sequence which shares at least 65%, 70%, 75%, 80%, 85%, 90%, 95%, 96%, 97%, 98%, 99%, or more sequence identity to such a fragment. Generally, "sequence identity" as used herein refers to the sequence identity between two sequences as determined using the well-known BLAST alignment program with default parameters as appropriate for the type of sequences (*i.e.*, nucleotide or amino acid). Alternative programs are BLASTN and BLASTP; details of these programs are known in the art and can be found at the website of the NCBI (National Center for Biotechnology Information).

In some embodiments, "soluble CD83" or "sCD83" as used herein refers to an epitope comprising at least a portion of the extracellular domain of a member of the CD83 family of proteins wherein said epitope stimulates an immune response to itself, *e.g*., when administered to a subject. In such embodiments, the immune response against the epitope which is sCD83 may mimic the activities and effects described herein for sCD83. That is, the immune response against the epitope which is sCD83 may have the activity of an sCD83 which comprises the extracellular domain of native CD83, or the immune response against the epitope which is sCD83 may have an effect on a subject to which it is administered that is similar to the effect produced by administration of sCD83 comprising the extracellular domain of native CD83. In such embodiments, the activity or effect of CD83 in an assay or on a subject may be a result of the immune response against the epitope or it may result from the activity of CD83 itself, or both.

CD83 for use in the invention may be a dimer or multimer of CD83 protein. Dimerization or multimerization may be achieved through formation of one or more disulfide bonds between the cysteine residues present within the monomeric form of the CD83 protein (which are present, e.g., at positions 12, 27, 35, 100, 107, 129, and 163 of the native protein), or by means of a bifunctional linker molecule (*e.g*., a diamine, a dicarboxylic acid compound or the like) connecting same or different functional moieties (*e.g*., carboxy groups, amino group, hydroxy groups, thio groups, *etc*.) within the monomeric form of the CD83 protein. The latter also includes the use of polypeptide linkers (*e.g*., out of small polar amino acid residues such as-[(Gly)ₓSer)_{y}- (where x is, *e.g.,* 3 or 4 and y is, *e.g.,* 1 to 5)) to yield dimeric structures which can directly be produced by recombinant techniques. In some embodiments, CD83 for use in the invention is a homodimer or homomultimer (*e.g*., sCD83 comprising amino acid residues 20 to 144 of the native CD83 protein (*i.e*., SEQ ID NO:2) connected via a disulfide bond between the fifth cysteine residue of the soluble CD83 (i.e., the cysteine residue corresponding to amino acid 129 in the native protein).

In some embodiments, CD83 may be a monomer; in such embodiments, one or more of the native cysteine residues of CD83 may be replaced by another amino acid residue, *e.g*., a small and/or polar amino acid residue. In some embodiments, preferably the small and/or polar amino acid residues are selected from serine, alanine, glycine, valine, threonine, *etc*. Where CD83 is a monomer, in some embodiments, at least one cysteine residue of the CD83 protein has been replaced, such as, *e.g*., the cysteine residue corresponding to position 129 of the full-length native protein may be replaced with alanine.

In some embodiments, the term "soluble CD83" or "sCD83" also encompasses fusion proteins of at least a portion of the extracellular domain of CD83 and functional fragments and derivatives (see, *e.g*., WO 2004/046182). Suitable fusion proteins include at least a portion of the extracellular domain of CD83 and at least one other peptides or protein, so long as the fusion protein retains CD83 activity as defined herein. Thus, in some embodiments, the fusion protein will comprise at least a portion of the extracellular domain of CD83. Such proteins or peptides are known in the art. In some embodiments, proteins comprising additional residues of CD83 outside the extracellular domain are encompassed by the term "soluble CD83." Thus, suitable derivatives include, but are not limited to, proteins having additional sequences attached to the C- or N-terminus, *e.g*., those currying part of a transmembrane domain at their C-terminus or carrying at the N-terminus a short peptide (*e.g*., Gly-Ser-Pro-Gly, which can result from cleavage of a thrombin site by thrombin, *e.g.,* as a product of an engineered fusion protein; or a short peptide resulting from a fragment of GST following cleavage of a fusion protein). In some embodiments, sCD83 consists of or includes amino acids 20 to 145 of the native, full-length protein (SEQ ID NO:2), which includes the extracellular domain and an additional amino acid at the C-terminus. In other embodiments, sCD83 consists of amino acids 20-143 of SEQ ID NO:2.

The activity of CD83 for use in the present invention can be evaluated by methods known in the art. A CD83 protein is said to have "CD83 activity" if it exhibits at least one activity of the native, full-length CD83 protein as measured by any suitable assay. A CD83 protein is said to have "CD83 activity" if in such an assay it has at least 10%, 20%, 30%, 40%, 50%, 60%, 70%, 80%, 90% or more of the activity of the native, full-length CD83 protein as measured in the same assay. Preferably, CD83 protein for use in the present invention is capable of binding to mature immunostimulatory dendritic cells and decreasing the ability of these dendritic cells to stimulate T-cell proliferation. In some embodiments, CD83 will repress or decrease TNF-α in mature DCs or monocytes, for example, in assays described in Example 9. This activity can be evaluated directly or indirectly and can be measured *in vivo* or *in vitro*; suitable assays are known in the art and include, for example, a mixed lymphocyte reaction assay, or "MLR" (see, *e.g*., Kruse et al. (2000) J. Virol. 74: 7127-7136; Lechmann et al. (2001) J. Exp. Med. 194: 1813-1821). Thus, CD83 protein for use in the invention can decrease the ability of mature immunostimulatory dendritic cells to stimulate T-cell proliferation by at least 10%, 20%, 30%, 40%, 50%, 60%, 70%, 80%, or 90% or more in comparison to an appropriate control such as, for example, an interaction between mature immunostimulatory dendritic cells and T-cells in the absence of sCD83. In some embodiments, CD83 is an epitope and has the activity of stimulating an immune response. This activity may be assayed by identifying anti-CD83 antibodies produced in response to the CD83 in an *in vitro* assay or following administration to a subject.

In some embodiments, CD83 protein for use in the invention can decrease the expression of TNF-α, CD80 and/or CD83 by immunostimulatory dendritic cells matured *in vitro* in the presence of soluble CD83 during at least one step of the maturation process by at least 10%, 20%, 30%, 40%, 50%, 60%, 70%, 80%, or 90% or more in comparison to an appropriate control (see, *e.g.,* Lechmann et al. (2001) J. Exp. Med. 194: 1813-1821; WO 2004/046182; Example 9). In this manner, the CD83 can be said to have changed the expression of cell surface markers, *i.e*., to have altered the immunophenotype of the treated cells. It is understood by those of skill in the art that commonly-used techniques for the analysis of expression of cell surface markers (*e.g*., FACS analysis) can sometimes yield data that represents mixed populations of cells, and care should be taken to identify which populations may be represented in a particular data set.

Other members of the CD83 family of proteins can be obtained by hybridizing a nucleic acid comprising, for example, all or a part of the human CD83 coding region that encodes the extracellular portion to various sources of nucleic acids (*e.g*., genomic DNA, cDNA, or RNA) from other animals, such as mammals, or from other tissues of the same organism. CD83 and other proteins used in the methods and compositions of the invention may be produced and purified by any suitable method. Methods for producing, purifying, and manipulating various proteins and derivatives thereof as well as nucleic acids encoding these are also well known in the art. See, *e.g.,* Sambrook et al. (1989) Molecular Cloning: A Laboratory Manual (Cold Spring Harbor Laboratory, N.Y.); see also U.S. Pat. No. 7,169,898; U.S. Pat. App. No. 10/382,397; and U.S. Pat. App. No. 10/535,522.

In some embodiments, CD83 is administered to a subject by providing to the subject a nucleic acid that encodes a CD83 protein. For example, CD83 could be administered to a subject as a DNA fragment comprising nucleotides 58 to 432 of SEQ ID NO:1 of U.S. Pat. No. 7,169,898, or as the corresponding mRNA (see, *e.g.,* U.S. Pat. No. 7,015,204). Similarly, in some embodiments, CD83 is provided to cells *in vitro* or *in vivo* by transforming a host cell (*i.e.,* a cell) with a nucleic acid that encodes a CD83 protein; the transformed host cell can then express CD83 protein, thereby providing CD83 protein to other cells as well as to itself *(i.e.,* the transformed host cell). In such embodiments, suitable host cells include dendritic cells. In embodiments where CD83 is provided as a nucleic acid encoding CD83 protein, the term "CD83" may also refer to the nucleic acid encoding the CD83 protein. Any suitable nucleic acid may be used, including DNA, RNA, or a synthetic nucleic acid, so long as it encodes a CD83 protein. A nucleic acid may be provided in a suitable vector for expression of the encoded protein or protein fragment. Suitable vectors and methods for producing them are known in the art.

As used herein, "therapeutic composition" refers to an exogenous composition which is used for therapeutic purposes and to which an immune response is undesirable. As such, the term "therapeutic composition" encompasses pure preparations *(i.e.,* preparations consisting of a single, essentially pure substance or compound) as well as mixtures of substances or compounds. As used herein, the term "therapeutic purposes" includes an effort to prevent, cure, or reduce or alleviate at least one symptom of a disease or disorder. In this manner, "therapeutic purposes" include both therapeutic and prophylactic uses of the compositions and/or methods of the invention; accordingly, therapeutic compositions include those used to prevent the development of a disease or disorder caused by the dysfunction or undesired function of an immune response involving T cells.

Suitable therapeutic compositions for use in the invention include, but are not limited to: antibodies, proteins, "small molecules," gene therapy vectors and any protein expressed by a gene therapy vector, antigens, allergens, cells (including stem cells), and tissue transplants. It is understood that these categories are not necessarily mutually exclusive; thus, for example, many proteins are antigens, and many antigens are proteins. In embodiments where the therapeutic composition is coadministered with CD83 but the subject is not also treated with at least one other therapeutic composition or at least one other immunosuppressive compound, the therapeutic composition is not a transplanted tissue. In some embodiments where the therapeutic composition is coadministered with CD83 but the subject is not also treated with at least one other therapeutic composition or at least one other immunosuppressive compound, the therapeutic composition is not an antigen associated with a transplanted tissue.

A suitable therapeutic composition which is an antigen is any composition to which an immune response may be raised *(i.e.,* any composition to which a subject does or could have an immune response). Suitable antigens include proteins and protein fragments, such as, for example, an antigen associated with an autoimmune disease to which tolerance is desired, such as an antigen which is involved in or associated with the development of an autoimmune disease or to which antibodies are found in a subject having that autoimmune disease.

Thus, exemplary antigens include myelin basic protein (which is involved in the development of multiple sclerosis), desmoglein-3 protein (which is believed to be involved in the development of Pemphigus vulgaris), thyroid stimulating hormone (TSH) receptor (which is involved in the development of Grave's disease), thyroid peroxidase (which is involved in the development of Hashimoto's thyroiditis), acetylcholine receptor (which is involved in the development of myasthenia gravis) and insulin (which is involved in the development of Type I diabetes mellitus). A suitable therapeutic composition which is an antibody may be any type of antibody or derivative thereof, including a polyclonal antibody, a monoclonal antibody, a single-chain antibody, or a portion or fragment of an antibody.

The methods described herein can induce tolerance even where the antigen to which an immune response is or may be directed is unknown, so long as the therapeutic composition used in the methods directs tolerogenic cells to the vicinity of the undesired immune response. Thus, suitable therapeutic compositions include antigens which are associated with a particular tissue to which tolerization is desired, regardless of the identity of the antigen in or near that tissue to which an undesired immune response is or may be directed. In this manner, in some embodiments, the methods and compositions disclosed may be particularly useful in treating and/or preventing diseases or disorders in which "determinant spreading" has occurred or might occur (see, *e.g.,* Dai et al. (2005) Cell. Mol. Immunol. 2: 169-175; Prat and Antel (2005) Curr. Opin. Neurol. 18: 225-230).

An antigen is "associated with" a particular tissue if it is expressed by cells of which that tissue is comprised, or if it is found on or near cells of which that tissue is comprised (e.g., in the extracellular matrix of that tissue). The use of an antigen "associated with" a particular tissue can involve the use of the antigen in purified form, separate from the tissue itself, or it can involve the use of the antigen *in situ* (*i.e.,* in the tissue). The term "tissue" as used herein encompasses discrete organs (*e.g*., liver, kidney, heart, lung, *etc.*) as well as "liquid" tissues (*e.g.,* blood, blood components such as plasma, cells such as T cells, *etc.*) and portions and subparts of either or both.

Where appropriate, therapeutic compositions may be selected to lack biological activity other than their ability to tolerize a subject when used in the methods described in the invention. Thus, for example, a therapeutic composition may consist of only a portion or fragment of the native insulin protein which lacks the normal physiological activity of native insulin. A suitable therapeutic composition which is an antibody may be any type of antibody or derivative thereof, including a polyclonal antibody, a monoclonal antibody, a single-chain antibody, or a portion or fragment of an antibody. One of skill in the art will be able to identify and provide suitable therapeutic compositions for use in the methods of the invention.

Suitable therapeutic compositions may include antigens or other agents which serve to stimulate tolerization whether or not they are associated with a particular tissue to which tolerization is desired, so long as an object of the invention is achieved *(e.g.,* tolerization to a transplanted tissue). A suitable therapeutic composition comprises total RNA or a subset of RNA isolated from a tissue of interest; this can be administered directly to the subject (see, *e.g*., U.S. Pat. No. 7,015,204) or can be used to transduce dendritic cells, which can then be used, for example, to treat a subject or for *in vitro* production of regulatory T cells. A therapeutic composition that is a protein or polypeptide may be administered as a protein or polypeptide or it may be administered by providing a nucleic acid encoding it to a cell or to a subject. A therapeutic composition may comprise RNA (see, *e.g*., U.S. Pat. No. 7,015,204). In some embodiments, more than one therapeutic composition is administered to a subject.

The term "immunosuppressive compound" refers to a compound which is capable of depleting the size of a population ofT and/or B clones of lymphocytes or which is capable of suppressing their reactivity, expansion, or differentiation. Immunosuppressive compounds for use in the methods of the invention include, but are not limited to: calcineurin inhibitors, including cyclosporine (also known as "CsA," marketed as Neoral® or Sandimmune®) and tacrolimus (also known as "FK506," marketed as Prograf®); purine metabolism inhibitors such as mycophenolate mofetil (also known as "MMF," marketed as Cellcept®) and azathioprine (marketed as Azasan® or Imuran®); proliferation inhibitors such as everolimus (marketed as Certican®) and sirolimus (also known as "rapamycin" or "Rapa," marketed as Rapamune®); monoclonal antibodies ("mAb"), such as anti-CD45 and anti-CD45RB (see, *e.g*., U.S. Pat. No. 7,160,987); monoclonal antibodies directed against T-cells, such as OKT3; monoclonal antibodies directed against the IL-2 receptor, including humanized anti-T_{aT} antibodies, such as basilixamab and daclizumab; substances which block T-cell co-stimulatory pathways, such as CTLA-4-Ig1 fusion protein; substances which are able to induce chimerism (*i.e*., the coexistence of donor and recipient immune cells, in which graft tissue is recognized as self); and non-myeloblative pre-transplantation treatments such as cyclophosphamide (marketed as Cytoxan®). For a discussion of immunosuppressives and their targets, see, *e.g*., Stepkowski (2000) Expert Rev. Mol. Med. June 21, 2000: 1-23.)

Sirolimus (also known as "rapamycin" or "Rapa," marketed as Rapamune®) is currently indicated for the prophylaxis of organ rejection in renal transplant patients aged 13 years and older. The manufacturer recommends that Rapamune® should be used initially in a regimen with cyclosporine and corticosteroids. Studies in experimental models (*i.e.,* mice, pigs, and/or primates) showed that sirolimus prolonged allograft survival, for example, of kidney, heart, skin, islet, small bowel, pancreatico-duodenal tissue, and bone marrow. Sirolimus reversed acute rejection of heart and kidney allografts in rats and prolonged the graft survival in presensitized rats. In some studies, the immunosuppressive effect of sirolimus lasted up to 6 months after discontinuation of therapy. The tolerization effect of sirolimus is allogenic-specific. In rodent models of autoimmune disease, sirolimus suppressed immune-mediated events associated with systemic lupus erythematosus, collagen-induced arthritis, autoimmune (Type I) diabetes, automyocarditis, experimental allergic encephalomyelitis, graft-versus-host disease, and autoimmune uveoretinitis.

Mycophenolate mofetil (also known as "MMF," marketed as Cellcept®) is currently indicated for the prophylaxis of organ rejection in patients receiving allogeneic cardiac (heart) or hepatic (liver) transplants. The manufacturer recommends that Cellcept® be used concomitantly with cyclosporine and corticosteroids. Mycophenolate mofetil has been demonstrated in experimental animal models to prolong the survival of allogeneic transplants including kidney, heart, liver, intestine, limb, small bowel, pancreatic islets, and bone marrow. Mycophenoloate mofetil was also shown to reverse ongoing acute rejection in the canine renal model and rat cardiac allograft model and inhibited proliferative arteriopathy in experimental models of aortic and cardiac allografts in rats as well as primate xenografts. Mycophenoloate mofetil has also been demonstrated to inhibit tumor development and prolong survival in murine tumor transplant models. Mycophenoloate mofetil may be used alone or in combination with other immunosuppressive agents.

Tacrolimus (also known as "FK506," marketed as Prograf®) is currently indicated for the prophylaxis of organ rejection in patients receiving allogeneic liver, kidney or cardiac transplants. Tacrolimus should be used concomitantly with adrenal corticosteroids. In heart transplant recipients, it is recommended that Prograf® be used in conjuction with azathioprine or MMF. Tacrolimus has been shown to prolong the survival of the host and of the transplanted graft in animal transplant models of liver, kidney, heart, bone marrow, small bowel and pancreas, lung and trachea, skin, cornea and limb. In animals, tacrolimus has been demonstrated to suppress some humoral immunity and, to a greater extent, cell-mediated reactions such as allograft rejection, delayed-type hypersensitivity, collagen-induced arthritis, experimental allergic encephalomyelitis and graft versus host disease. While the invention is not bound by the mechanism of action of any of the compounds or their combinations, and although the exact mechanism of action of tacrolimus is not known, tacrolimus is believed to inhibit the phosphatase activity of calcineurin and to inhibit T-lymphocyte activation.

Cyclosporine (also known as "CsA," marketed, *e.g.,* as Neoral® or Sandimmune®) is currently indicated for prophylaxis of organ rejection for allogeneic transplants of kidney, liver, and heart. Cyclosporine has been used in combination with azathioprine and corticosteroids, and can be used in combination with methotrexate in rheumatoid arthritis patients. Cyclosporine is indicated for the treatment of patients with severe active rheumatoid arthritis where the disease has not adequately responded to methotrexate. Cyclosporine is also indicated for the treatment of adult, nonimmunocompromised patients with severe recalcitrant plaque psoriasis. Cyclosporine is a potent immunosuppressive agent that has prolonged survival of allogeneic transplants involving skin, kidney, liver, heart, pancreas, bone marrow, small intestine and lung in animal models. Cyclosporine has been shown to suppress some humoral immunity and, to a greater extent, cell-mediated immune reactions such as allograft rejection, delayed hypersensitivity, experimental allergic encephalomyelitis, Freund's adjuvant arthritis and graft-versus-host disease in many animal species for a variety of organs. While the invention is not bound by the mechanism of action of any of the compounds or their combinations, cyclosporine has been shown to inhibit immunocompetent lymphocytes in the G0 and G1 phase of the cell cycle and to preferentially inhibit T-lymphocytes.

By "effective amount" of a substance is intended that the amount is at least sufficient to achieve at least one object of the invention when administered to a subject according to the methods disclosed in the invention. Thus, for example, an "effective amount" of a therapeutic composition is at least sufficient to tolerize the subject to the therapeutic composition when it is coadministered to a subject with CD83 and at least one other immunosuppressive compound. Similarly, an "effective amount" of CD83 is at least sufficient to tolerize the subject to a therapeutic composition when it is coadministered with a therapeutic composition and at least one other immunosuppressive compound. An "effective amount" of an immunosuppressive compound is at least sufficient to produce a measurable effect on at least one symptom of a disease or disorder caused by the dysfunction or undesired function of an immune response when it is coadministered with CD83 and, optionally, a therapeutic composition. In some embodiments, an effective amount of an immunosuppressive compound is at least sufficient to induce tolerance and/or immunosuppression as defined elsewhere herein. The effective amount of an immunosuppressive compound may be determined with regard to symptoms exhibited an individual subject or it may be determined from clinical studies or extrapolated from appropriate studies in model systems. Thus, for example, an effective amount of an immunosuppressive compound includes an amount that would be expected to produce a measurable effect on at least one symptom of a disease or disorder based on a dosage range determined in a clinical study utilizing a method of the invention.

An effective amount can be administered in one or more administrations, applications or dosages. Suitable administrations, applications, and dosages will vary depending on a number of factors, including but not limited to: specific activity of the compositions; the formulation of the compositions; the body weight, age, health, disease and condition of the subject to be treated; and the route of administration of the compositions into the subject. In some instances, the minimum amount of CD83 required to be an effective amount may be reduced due to the presence in the patient of pre-existing CD83, particularly soluble CD83 (see, *e.g.,* Hock et al. (2006) (Tissue Antigens 67: 57-60)); one of skill in the art will readily be able to adjust the dosage and administration, *etc.,* in order to achieve the best results. For example, CD83 may be administered to a patient within a range having: a lower end of 0.01, 0.05, 0.1, 0.5, 1, 2, 5, 7, 10, 20, 50, 70, 100, 200, 500, or 700 mg/kg, or 1, 2, 5, 7, 10, 20, 50, or 100 g/kg; and an upper end of 0.05, 0.1, 0.5, 1, 2, 5, 7, 10, 20, 50, 70, 100, 200, 500, or 700 mg/kg, or 1, 2, 5, 7, 10, 20, 50, 100, or 200 g/kg.

In some embodiments, the methods disclosed in the invention can induce tolerance even when the antigen to which an immune response is or may be directed is unknown. Thus, suitable therapeutic compositions include antigens which are associated with a particular tissue to which tolerization is desired, regardless of the identity of the antigen in or near that tissue to which an undesired immune response is or may be directed. In this manner, in some embodiments, the methods and compositions of the invention may be particularly useful in treating and/or preventing diseases or disorders in which "determinant spreading" has occurred or might occur (see, *e.g.,* Dai et al. (2005) Cell. Mol. Immunol. 2: 169-175; Prat and Antel (2005) Curr. Opin. Neurol. 18: 225-230). An antigen is "associated with" a particular tissue if it is expressed by cells that the tissue comprises, or if it is found on or near cells that the tissue comprises (*e.g*., in the extracellular matrix of that tissue).

The methods provided by the invention provide the benefit of a synergistic effect produced by the coadministration of CD83 with at least one other immunosuppressive compound, such that the efficacy of the combined treatment is much higher than would be expected from combining the two individual treatments. Moreover, if CD83 is coadministered to a subject with two or more other immunosuppressive compounds, even greater benefits can be provided. In this manner, the invention provides combination (or "coadministered") treatments which have greater efficacy than is provided by the individual treatments alone. In some embodiments, the combination or coadministered treatment provides greater benefit than the sum of the benefits provided by each individual treatment. In some embodiments, the combination or coadministered treatment provides a benefit that is greater than the benefit provided by individual treatment by at least 10%, 20%, 25%, 30%, 50%, 75%, 100%, 200%, or more, or by 1.5-fold, 2-fold, 3-fold, 4-fold, or more. In some embodiments, the combination or coadministered treatment provides a benefit that is greater than the sum of the benefits provided by each individual treatment by at least 10%, 20%, 25%, 30%, 50%, 75%, 100%, 200%, or more, or by 1.5-fold, 2-fold, 3-fold, 4-fold, or more.

This improved efficacy of the combination (or "coadministered") treatment provides the methods of treatment of the invention, in which lower doses of an individual compound can be used to treat a subject than would be required if that compound were used alone. In this manner, the invention makes possible effective treatment using less of each individual compound than is currently used. In other words, the invention provides methods of using reduced amounts of compounds for treating a subject and methods of reducing the amount of a compound used to treat a subject. Thus, the invention further provides a treatment with a lower probability of side effects and/or a reduced severity of side effects due to the lowered dosages used. Such side effects include those seen generally with calcineurin inhibitors (*e.g*., nephrotoxicity). Individual calcineurin inhibitors also tend to produce particular side effects; for example, side effects that tend to be seen with cyclosporine treatment include diabetes, hypertension, hyperlipidemia, tremor, gum hyperplasia hirsutism, and skin changes, while side effects that tend to be seen with tacrolimus treatment include an increased incidence diabetes, but less hyperlipidemia, hypertension, and gum hyperplasia than tend to be seen with cyclosporine treatment. Side effects commonly seen with mycophenolate mofetil treatment include an increased risk of leucopenia, diarrhea, anemia, hypertension, esophagitis, gastritis and gastrointestinal hemorrhages.

By "lower doses" is intended that an individual compound is administered to a subject at dosage levels below those that would otherwise be used to treat a subject. In some embodiments, by "lower doses" is intended that an individual compound is administered to a subject at dosage levels below those recommended by the manufacturer, *i.e.,* dosage levels recommended in the package insert for the compound. For reference, dosage levels presently recommended in the package inserts for several compounds of interest are as follows. Where a recommended dosage level varies by stage of therapy or treatment interval (*e.g*., dosage levels for new transplant patients versus maintenance dosage levels), the methods of the invention include methods of treatment in which the subject is treated with lower doses of a compound during only one stage of therapy or treatment interval, or during at least one stage of therapy or treatment interval. Stages of therapy and/or treatment intervals are understood by those of skill in the art and include, for example: long-term maintenance or management of a chronic autoimmune disorder or disease; pre-transplant preparation; post-transplant recovery; and post-transplant maintenance. Alternatively, a treatment interval may be a period of time during which treatment is conducted and/or evaluated, such as a day, a week, a month, or two, three, four, five, or six months, or a year, or an interval during or following which the subject is shown to have achieved certain treatment landmark(s).

For tacrolimus (also known as "FK506," marketed as Prograf®), the manufacturer recommendation is for dosing no sooner than 6 hours after transplantation at a starting dose of 0.01 mg/kg/day (for heart transplants) or 0.03-0.05 mg/kg/day (for liver or kidney transplants) as a continuous intravenous infusion. Continuous infusion should be continued only until the patient can tolerate oral administration.

For sirolimus (also known as "rapamycin," marketed as Rapamune®), the recommended dosing varies depending on the immunologic risk to the patient and the stage of post-transplantation treatment. For patients at low to moderate immunologic risk, it is recommended that Rapamune® oral solution and tablets be used in a combination with cyclosporine and corticosteroids; cyclosporine should be withdrawn 2-4 months after transplantation. For *de novo.* transplant recipients, a loading dose of Rapamune® corresponding to 3 times the maintenance dose should be given. For example, for renal transplant patients, a maintenance dose of 2 mg is recommended along with a loading dose of 6 mg. Patients considered for cyclosporine withdrawal should be receiving Rapamune® and cyclosporine combination therapy. At 2 to 4 months following transplantation, cyclosporine should be progressively discontinued over 4 to 8 weeks and the Rapamune® dose should be adjusted to obtain whole blood trough levels within the range 16 to 24 ng/mL for the first year following transplantation; thereafter the target should be 10-20 ng/mL.

For patients at high immunologic risk, it is recommended that Rapamune® oral solution and tablets be used in a combination with cyclosporine and corticosteroids for the first year. For patients receiving Rapamune® with cyclosporine, Rapamune® therapy should be initiated with a loading dose of up to 15 mg on the first day following transplantation. Beginning on the second day after transplantation, an initial maintenance dose of 5 mg/day should be given. The starting dose of cyclosporine should be up to 7 mg/kg/day in divided doses and the dose should subsequently be adjusted to achieve target whole blood trough concentration (*i.e*., minimum concentration in the blood). Prednisone should also be administered, at a minimum of 5 mg/day.

For use in renal allograft recipients, the initial dose of Rapamune® should be administered as soon as possible after transplantation. Frequent Rapamune® dose adjustment based on non- steady-state Rapamune® concentrations can lead to overdosing or underdosing, so dosing adjustments should be made and monitored carefully. Once the maintenance dose is adjusted, patients should be kept on the new maintenance dose for at least 7 to 14 days.

For mycophenolate mofetil (also known as "MMF," marketed as Cellcept®), the manufacturer recommendation for renal transplant patients is for a dose of 1 gram administered orally or intravenously (over no less than 2 hours) twice a day for a total daily dose of 2 grams. For pediatric patients between 3 months and 18 years of age, the recommended daily dose of Cellcept® oral suspension is 600 mg/m² twice daily (up to a maximum of 2 grams/ 10 ml oral suspension). Body surface area may be determined, for example, using standard tables of body surface area as a function of weight (see, *e.g.,* Sharkey et al. (2001) British J. Cancer 85: 23-28). For heart transplant patients, adults should be treated with a dose of 1 gram administered intravenously twice a day ("*b.i.d.*") over no less than 2 hours or 1.5 gram administered orally twice a day for a daily dose of 3 grams. For hepatic transplant patients, adults should be treated with a dose of 1 gram administered intravenously *b.i.d.* over no less than 2 hours or a dose of 1.5 gram administered orally *b.i.d.* for a total daily dose of 3 grams.

For cyclosporine (also "CsA," marketed as Neoral® or Sandimmune®), the manufacturer recommendation is that an initial oral dose of Neoral® can be given 4-12 hours prior to transplantation or postoperatively. In new transplant patients, the mean initial dose (± SD) is 9 (± 3) mg/kg/day for renal transplant patients, 8 (± 4) mg/kg/day for liver transplant patients and 7 (± 3) mg/kg/day for heart transplant patients. Total daily doses are divided into two equal doses, and the dose is subsequently adjusted to achieve a pre-defmed cyclosporine blood concentration; a representative dosage schedule based on a person's weight starts at 2 mg/kg/day for the first 14 days; thereafter, the dosage is tapered to 1 mg/kg/day by one week, further tapered to 0.6 mg/kg/day by two weeks, to 0.3 mg/kg/day by one month, and to 0.15 mg/kg/day by two months and thereafter as maintenance therapy. Initially, adjunct therapy with adrenal corticosteroids is recommended.

For treatment of rheumatoid arthritis, the initial dose of cyclosporine is 2.5 mg/kg/day taken twice daily as a divided oral dose. Other treatments of salicilates, nonsteroidal antiinflammatory agents, and oral corticosteroids may be continued. To control adverse events at any time, doses should be decreased by 25-50%. For treatment of psoriasis, the initial dose of cyclosporine is 2.5 mg/kg/day taken twice daily as a divided oral dose. Barring adverse events, patients should be kept at this dose for at least 4 weeks. If significant clinical improvement is not seen after 4 weeks, the dose can be increased at 2 week intervals by 0.5 mg/kg/day to a maximum of 4 mg/kg/day. To control adverse events at any time, doses should be decreased by 25-50%.

The terms compound, "composition," and "subatance" as used herein each encompass any substance intended for use in a method disclosed in the invention (*i.e*., including CD83, a therapeutic composition, or an immunosuppressive compound). A substance is "coadministered" with another substance and "coadministration" occurs when substances are simultaneously administered to the subject, or when a substance is administered to the subject within 2, 4, 6, 8, 10, 12, or 14 hours, or within 1, 2, 3, 4, 5, 6, 7, 8, 9, 10, 15, 20, 25, 30, 35, 40, 45, 50, 60, 70, 80, 90, 100, 150, or 200 days before or after another substance is administered to the subject. When substances are simultaneously administered, they can be combined prior to administration, or separately administered at the same time.

The methods provided in the invention can include one dose or administration or multiple doses or administrations of each compound or composition. In embodiments where multiple administrations are performed, at least one administration of a compound or composition will occur within the "window of effectiveness" of another compound or composition that is administered. In some embodiments, administration of a second substance is deemed to have occurred within the "window of effectiveness" of a first substance if the efficacy of the combined treatment (*i.e*., of the administration of both substances) is or is expected to be greater than or different from one or both individual treatments if they had been administered separately, *i.e*., in an appropriate control experiment. In some embodiments, administration of a second substance is deemed to have occurred within the "window of effectiveness" of a first substance if the efficacy of the combined treatment *(i.e.,* of the administration of both substances) is or is expected to be greater than or different from the combined efficacies of the individual treatments if they had been administered separately, *i.e.,* in an appropriate control experiment. For example, a subject may be tolerized to a therapeutic composition by a method comprising administration of CD83 and administration of the therapeutic composition within the window of effectiveness of CD83; this result would not be expected if the subject was treated separately with CD83 and with the therapeutic composition, such that administration of the therapeutic composition occurred outside the window of effectiveness of CD83.

In some embodiments, at least one compound or composition is administered so that there is at least some overlap of that substance's "window of effectiveness" with the "window of effectiveness" of at least one other substance. Such an overlap is deemed to have occurred if the efficacy of the combined treatment is or is expected to be greater than or different from the combined efficacies of the individual treatments if they had been administered separately, *i.e*., in an appropriate control experiment. In some embodiments, during a particular course of treatment, additional administrations may occur before, during, or after the window of effectiveness of one or more substances, while in other embodiments, there are no additional administrations outside the window of effectiveness of one or more substances. In some embodiments, all of the administrations of each substance occur within the window of effectiveness of each other substance administered to the subject. In embodiments where more than two substances are administered to a subject, a substance can be administered within the window of effectiveness of one, two, three, four, five, six, seven, eight, nine, ten, or more other substances.

The methods described in the invention can also include administration of more than one therapeutic composition. Thus, the methods can include administration of multiple therapeutic compositions. The therapeutic compositions may be similar or dissimilar. For example, therapeutic compositions that were administered in a method provided by the invention to a subject could include related antigens. Thus, for example, therapeutic compositions administered to a subject could comprise RNA prepared from a particular tissue ("total tissue RNA").

Because the subject can be tolerized to exogenous compounds by CD83, for therapeutic purposes, it is important that the subject not be exposed at least during treatment, or at least during the window of effectiveness of CD83, to compounds to which tolerization is not desired. Thus, for example, the subject should not be exposed to tumor-specific antigens and disease-causing microorganisms including viruses, bacteria, mold, *etc*. Generally, as used herein, by "subject" is intended any animal in need of treatment. Thus, for example, a "subject" can be a human patient or a non-human mammalian patient or may be another patient which is an animal.

Where prevention of a disease or medical condition is desired, a subject may be treated at regular intervals (*e.g*., approximately: every two years, every year, every six months, every two to four months, or every month). However, in all embodiments of the invention, the methods and compositions are administered to a subject that has been identified as having a particular disease or disorder caused by the dysfunction or undesired function of an immune response or to a subject that has been identified as being likely to develop a particular disease or disorder. For example, a subject may be identified as likely to develop such a particular disease or disorder as a result of examination of the subject's family history, of a medical test such as a genetic test or a test to determine the subject's enzyme or metabolite levels, or of being diagnosed with another disease or disorder. In this manner, for example, the methods of the invention may be used to treat an autoimmune disease, to prevent the development of an autoimmune disease, or to reduce the risk of a subject for developing an autoimmune disease. Generally, a course of treatment ends when the subject is no longer being treated to alleviate or prevent a particular disease or medical condition caused by the dysfunction or undesired function of an immune response.

Thus, the invention provides a method of treatment or prevention of a disease or medical condition caused by the dysfunction or undesired function of an immune response, wherein an effective amount of CD83 is coadministered to a subject along with' at least one other immunosuppressive compound so that immunosuppression is achieved. Optionally, a therapeutic composition may also be administered. If a therapeutic composition is administered, it is administered to the subject during the window of effectiveness of CD83 so that the subject is tolerized to the therapeutic composition. Any form of administration may be chosen for delivery of CD83 and the therapeutic composition so long as this objective is achieved. Thus, for example, CD83 and the therapeutic composition may be administered together and via the same form of administration or they may be administered separately and/or via different forms of administration. Thus, for example, CD83 may be administered to the subject as a protein or it may be administered to the subject as a nucleic acid encoding CD83 (e.g., as an mRNA or in an expression vector).

The methods provided in the invention can be employed in order to treat subjects that are or may become affected by a disease or disorder caused by the dysfunction or undesired function of an immune response, such as, for example: an allergy; asthma; rejection of a tissue transplant; an immune response to a chronically administered substance; an autoimmune disease such as myasthenia gravis, multiple sclerosis, vasculitis, a chronic inflammatory bowel disease such as Crohn's disease or ulcerative colitis, ankylosing spondylitis, systemic lupus erythematosis, skin diseases such as psoriasis, rheumatoid arthritis, and insulin-dependent diabetes mellitus; and AIDS. The methods provided in the invention can be used to treat subjects afflicted by a disease or disorder which involves B-cells or B-cell functions, such as, for example: B-cell hyperplasias such as leukemia (including multiple myeloma and acute lymphoblastic leukemia); B-cell hyperactivity associated with AIDS; toxic shock syndrome; serum sickness; and periodontal disease (see, *e.g*., Mahanonda et al. (2002) J. Periodontal Res. 37: 177-183). Serum sickness is a group of symptoms caused by an undesired immune response to certain medications or antiserum. That is, serum sickness may result when antiserum from another animal or human is given to a subject in an effort to induce passive immunization. In some embodiments, the methods provided by the invention provide treatment for a disease or disorder which involves B-cells or B-cell functions, wherein the treatment does not result in B-cell depletion, *i.e*., a decrease in the number and/or subtype of B cells in the subject.

The compositions and methods provided in the invention can be used alone (*i*.*e*., they can be used to treat a subject that is not receiving other treatments) or they can be administered to a subject that is receiving or has received other treatments. For example, a subject afflicted with a particular disease or disorder could be treated only with the methods and compositions of the invention, or they could be treated with the methods and compositions described in the invention as well as another treatment or therapy commonly administered to subjects that are or may become affected by the particular disease or disorder, such as, for example, conventional chemotherapy or pharmaceutical treatment. Thus, for example, methods described in the invention can further comprise the use or coadministration of other compounds including non-immunosuppressive compounds. Thus, the methods provided by the invention can further comprise the use or coadministration of other compounds, including, for example, corticosteroids, anti-inflammatory agents such as, e.g., Azathioprine, and anti-metabolite drugs such as, e. g., methotrexate.

Thus, in some embodiments, the methods described in the invention are used to prevent, cure, or alleviate at least one symptom of rejection of a tissue transplant in a tissue recipient. In such embodiments, the transplant recipient ("recipient" or subject) may be treated with CD83 and at least one other immunosuppressive compound or at least one therapeutic composition. The therapeutic composition may be an antigen associated with the transplanted tissue, such as, for example, an antigen prepared from or extracted from the transplanted tissue, e.g., by lysis or homogenization of a sample of the transplanted tissue. Generally, treatment of transplant recipients according to the methods provided by the invention can include treatment prior to, in conjunction with (*i*.*e*., at the same time), and/or following the transplantation of the tissue. The therapeutic composition is the transplanted tissue itself and so no additional administration of therapeutic composition is made to the recipient other than the introduction of the transplanted tissue; in such embodiments, at least one other immunosuppressive compound or at least one therapeutic composition is also administered. The methods disclosed in the invention prevent, cure, or alleviate all adverse symptoms of rejection of a tissue transplant, such that continued treatment of the transplant subject to prevent rejection becomes unnecessary; in such embodiments, it is said that graft tolerance has been induced in the subject.

In some embodiments, the donor of the tissue to be transplanted ("transplant donor") may be treated (e.g., with CD83 and at least one other immunosuppressive and, optionally, at least one therapeutic composition according to the methods disclosed in the invention) prior to removing the tissue for transplantation in the intended recipient. Substances are administered to the transplant donor as described herein, but the object of the treatment is to induce tolerance and/or immunosuppression in the transplant recipient. Here, too, the therapeutic composition may be an antigen associated with the transplanted tissue (e.g., an organ). Both the transplant donor and the transplant recipient may be treated according to the methods disclosed in the invention.

The tissue to be transplanted is removed from the donor and then exposed to a solution containing CD83; that is, for example, the tissue may be stored in and/or perfused with a solution containing CD83 prior to transplantation. The tissue to be transplanted is removed from the donor and then exposed to a solution containing CD83 and at least one other immunosuppressive compound; that is, for example, the tissue may be stored in and/or perfused with a solution containing CD83 and the at least one other immunosuppressive compound. Therapeutic compositions may also be administered to the donor. As a result, when this tissue is then introduced into the transplant recipient, the CD83 and other immunosuppressive compound(s) in the tissue and on the surface of the tissue will be coadministered to the subject along with an antigen associated with the tissue. "Tissue" as used herein encompasses discrete organs and/or specialized tissues (*e*.*g*., liver, kidney, heart, lung, skin, pancreatic islets, etc.) as well as "liquid" tissues (e.g., blood, blood components such as plasma, cells such as dendritic cells, *etc*.); the term "tissue" also encompasses portions and subparts of discrete organs and "liquid" tissues.

Those of skill in the art are familiar with methods of assessment and treatment of transplant recipients and donors in order to achieve the best possible outcome for both recipient and donor. Thus, those of skill in the art will readily be able to assess and adjust dosages and administration of CD83, at least one other immunosuppressive compound, and optionally a therapeutic composition as appropriate for a particular subject. As will be readily appreciated from the discussion above, the methods disclosed in the invention encompass a number of steps; these steps can be performed in any order so long as at least one object of the invention is accomplished. Because the methods may involve multiple administrations of multiple compounds, some overlap of steps may also occur.

The methods and compositions disclosed in the invention provide tolerance to gene therapy vectors such as, for example, adeno-associated virus ("AAV") and vectors derived from an AAV, and/or to a gene product encoded by the gene therapy vector. Thus, for example, when gene therapy is administered to a subject that lacks a particular gene product (e.g., when a subject has a genetic defect resulting in the absence of a particular protein), that subject could be treated using the methods to prevent an unwanted immune response to the gene product when it is introduced into the subject. Preferably, gene therapy vectors used in the compositions and methods described in the invention will have no associated pathologies. In some embodiments, the methods and compositions of the method provide tolerance to substances which are administered to a subject repeatedly (*i.e*., "chronically") and to which an immune response is undesired, such as, for example, beta interferon, which is often administered to subjects afflicted with multiple sclerosis.

The methods and compositions provide dendritic cells ("DCs") which are "tolerogenic"; that is, they are capable of inducing immune tolerance (*i*.*e*., "tolerizing a subject") as described earlier. Tolerogenic activity of a dendritic cell can be evaluated directly or indirectly and can be measured *in vivo* or *in vitro;* suitable assays are known in the art and include, for example, the ability to produce regulatory T cells following incubation with a CD4⁺ T cell population *in vitro* and/or a detectable change in the expression of a cell surface marker such as CD14 or CD80 (see, e.g., Steinman et al. (2003) Ann. Rev. Immunol. 21: 685-711; Kubach et al. (2005) Int. J. Hematol. 81: 197-203).

The tolerance induced by a tolerogenic dendritic cell may be induced *in vivo* or *ex vivo* and may be specific to an antigen presented by the DC. The term "immunostimulatory" DCs is used herein to distinguish "normal" or native DCs from the tolerogenic DCs of the invention. Methods described in the invention to produce tolerogenic DCs may be performed *in vivo* or *ex vivo,* and comprise the step of exposing the cells to CD83 and at least one other immunosuppressive compound. The cells may be exposed to the CD83 and other immunosuppressive compound(s) during the maturation process or after the maturation process is complete, whether *in vivo* or *ex vivo* (*in vitro).* Where the maturation process is performed *in vitro,* by "exposed" is intended that the cells may be incubated in the presence of CD83 and the other immunosuppressive compound(s) or may be cultured (*i.e*., the cells may grow and divide) in the presence of CD83 and the other immunosuppressive compound(s). The cells may be exposed to CD83 and the other immunosuppressive compound(s) in any suitable manner; e.g., CD83 and the other immunosuppressive compound(s) may be added to the growth medium or, where possible, one or more of CD83 and the other immunosuppressive compound(s) may be expressed by cells in the culture following transfection with an RNA encoding them.

Methods are described to produce tolerogenic DCs comprise exposing immature DC to CD83 and at least one other immunosuppressive compound during the production process (*e*.*g*., during the final maturation step). Generally, in these methods, immature dendritic cells may be exposed to CD83 within a range having: a lower end of 0.01, 0.05, 0.1, 0.5, 1, 2, 3, 4, 5, 6, 7, 8, 9, 10, 12, 15, 20, 50, or 100 micrograms per ml of media; and an upper end of 0.05, 0.1, 0.5, 1, 2, 3, 4, 5, 6, 7, 8, 9, 10, 12, 15, 20, 50, 100, or 200 micrograms per ml of media. Most preferably, DC are matured in the presence of 1-10 µg/ml CD83. Dosages of the other immunosuppressive compound(s) can be readily selected by one of skill in the art. By "immature DC" as used herein is intended that the cells are capable of becoming mature dendritic cells if cultured appropriately (if *in vitro*) or if allowed to mature (if in *vivo*). Thus, the methods disclosed comprise exposing cells to CD83 during at least one step of the maturation process and exposing cells to the other immunosuppressive compound(s) during at least one step of the maturation process; these steps may be the same step(s) or they may be different and/or non-overlapping step(s). Cells are exposed to CD83 and/or the other immunosuppressive compound(s): throughout at least one step of the maturation process; during more than one step of the maturation process; or during the entire maturation process.

Tolerogenic dendritic cells may also be loaded with antigen for presentation by "pulsing" the cells with antigen or by transducing the cells with RNA (see, e.g., U.S. Pat. No. 6,387,701; U.S. Pat. No. 6,670,186; U.S. App. No. 10/362,715). The methods described may also be combined with other methods known in the art to provide additional advantages (see, e.g., U.S. Pat. No. 5,831,068; U.S. App. No. 11/246,387). It will be appreciated that the steps of the methods may be performed in any order which produces the desired result. Thus, for example, cells may be loaded with antigen prior to or after their exposure to CD83 and/or to the other immunosuppressive compound(s). In this manner, the methods include the production of tolerogenic dendritic cells by a method comprising the coadministration of CD83, at least one other immunosuppressive compound, and optionally a therapeutic composition (e.g., an antigen of interest) to cells during the maturation process.

Mature immunostimulatory dendritic cells generally exhibit a characteristic array of cell surface markers. Thus, generally, a mature immunostimulatory DC will exhibit no or little expression of CD 14. Also, generally, a mature immunostimulatory DC (whether a native DC produced *in vivo* or a DC produced *in vitro*) will exhibit characteristics such as, for example, expressing the cell surface markers CD80, CD83, and CD86 and secreting IL-12. In contrast, the tolerogenic DCs produced have a reduced level of expression of at least one of these characteristic cell surface markers. The level of expression of a particular cell surface marker on a tolerogenic DC produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same cell surface marker on an appropriate control cell (e.g., a native mature immunostimulatory DC, or a mature immunostimulatory DC obtained via *in vitro* maturation). A tolerogenic DC exhibits undetectable expression of CD83 and greatly reduced expression of CD80. A tolerogenic DC exhibits CD83 expression that is reduced by at least 95% and CD80 expression that is reduced by at least 50% for each marker in comparison to an appropriate control cell. A tolerogenic DC will exhibit higher expression of CD 14 than a mature immunostimulatory DC. Tolerogenic dendritic cells may be produced and/or assayed for function using techniques known in the art (see, e.g., WO 2004/046182), or as described in the experimental section or as follows.

For example, cells can be cultured using standard medium with 1% human plasma (e.g., RPMI 1640 (BioWhittaker, Verviers, Belgium) supplemented with glutamine (200 µg/ml, BioWhittaker, Verviers, Belgium), penicillin/streptomycin (20 µg/ml), 10 mM Hepes, pH 7.5 (Sigma-Aldrich), and 1% human plasma from a single donor (previously heat-inactivated by incubating at 56°C for 30 min.)). Human PBMCs can be isolated from buffy coats by sedimentation in Ficoll-Hypaque (Amersham Pharmacia Biotech, Freiburg, Germany), seeded onto 100 mm culture dishes coated with IgG (10 µg/ml γ-globulin from Cohn fraction; Sigma-Aldrich), and incubated at 37°C in 5% CO₂. After 1 hour and 7 hour incubations, non-adherent cell fractions are removed and the adherent cells further cultured in 1% human plasma medium supplemented with the cytokines GM-CSF (800 U/ml) and IL-4 (500 U/ml). On day 3 of the incubation period, fresh medium is added (including GM-CSF at 400 U/ml and IL-4 at 500 U/ml). On day 4 or 5, non-adherent cells are collected, counted, and transferred into new dishes at a density of 0.3-0.5 x 10⁵ cells/ml. For the final maturation step, 1% human plasma medium is supplemented with TNF-α (1.25 ng/ml), GM-C3F (40 U/ml), IL-4 (200 U/ml), prostaglandin E₂ (0.5 pg/ml; see, e.g., Lechmann et al. (2001) J. Exp. Med. 194: 1813-1821), and soluble CD83 (4 µg/ml). Alternatively, for the final maturation step, the maturation cocktail comprises IL-1β, TNF-α, PGE₂, and soluble CD83 (4 µg/ml). On day 8, cells can be analyzed by FACS. Cells matured by this method reveal a clear reduction in the cell surface expression of CD80 (e.g., from 96% to 66%) and CD83 (e.g., from 96% to 30%) and an increase in CD14 positive cells when compared with normally matured DCs (see, e.g., WO 2004/046182). Expression of other cell surface markers (e.g., MHC Class I and II) may not be significantly affected. In other embodiments, cells can be matured according to the protocol as described above but soluble CD83 can be added to the maturation cocktail after one, two, three, or more days.

Dendritic cells exposed to CD83 and the other immunosuppressive compound(s) can be compared to dendritic cells that were not exposed to CD83 and the other immunosuppressive compound(s) in various ways to evaluate their tolerogenic or immunostimulatory properties. Appropriate assays are known in the art (*e*.*g*., the MLR assay); some are also described in the experimental section. A typical feature of MLR assays is the formation of clusters of immunostimulatory DCs and proliferating T cells. Addition of soluble CD83 on day 1 of the assay strongly inhibits the typical cell cluster formation (see, e.g., Lechmann et al. (2001) J. Exp. Med. 194: 1813-1821). Furthermore, soluble CD83 inhibits the ability of mature dendritic cells to stimulate T cell proliferation in a concentration-dependent manner (see, *e.g.,* Lechmann *et al.* (2001), *ibid.*).

The tolerogenic dendritic cells are used to produce regulatory T cells (see, e.g., U.S. Pat. App. No. 10/661,804). Briefly, regulatory T cells may be produced from a population comprising CD4⁺ T cells and/or CD8⁺ T cells. These T cell populations may be isolated from a subject or may be cultured. Subpopulations of T cells may also be used, such as, for example, populations sorted by cell surface marker so as to comprise enriched populations of particular cells (*e*.*g*., CD4⁺ CD25⁺ T cells or CD4⁺ CD25⁻ T cells). The T cells are then cultured or incubated with tolerogenic dendritic cells *i.e.,* dendritic cells which have been exposed to sCD83 and at least one other immunosuppressive compound, for example, during the maturation process. If the regulatory T cells are being produced *in vitro,* the tolerogenic dendritic cells may be allogeneic to or syngeneic with the T cells. The tolerogenic DCs are loaded with antigen (*e*.*g*., pulsed with antigen or transfected with antigen-encoding RNA). The tolerogenic DCs may present the antigen to T cells.

During production of tolerogenic T cells, the T cells may be exposed to and/or cultured with the tolerogenic DCs once or more than once. For example, the T cells may be cultured with DCs for days or weeks; the DCs in the mixed culture may be replenished if necessary. Culture will continue until a therapeutic amount of regulatory T cells has been obtained. Other culture techniques and/or additives may be used to improve the results obtained; for example, the culture media may also contain cytokines such as IL-2.

Generally, regulatory T cells secrete IL-10 and/or TGF-β (see, *e.g*., Walsh et al. (2004) J. Clin. Invest. 114: 1398-1403); assays for confirming secretion of these cytokines are known in the art. In some embodiments, regulatory T cells will inhibit a mixed lymphocyte reaction by at least 10%, 20%, 30%, 40%, 50%, 75%, 90%, 95%, or 100%. Mixed lymphocyte reaction assays are well-known in the art. The regulatory T cells of the invention may be antigen-specific. CD4⁺ CD25⁺ regulatory T cells generally express characteristic cell surface markers including CD4, CD25, and Foxp3; assays for cell surface markers are well-known in the art.

The term "vector" refers to a plasmid, virus, or other vehicle known in the art that can be manipulated by insertion or incorporation of a polynucleotide. Such vectors can be used for genetic manipulation (*i*.*e*., "cloning vectors") or can be used to transcribe and/or translate the inserted polynucleotide ("expression vectors"). A vector generally contains at least an origin of replication for propagation in a cell and a promoter. Control elements present within an expression vector, including expression control elements as set forth herein, are included to facilitate proper transcription and translation (e.g., splicing signals for introns, maintenance of the correct reading frame of the gene to permit in-frame translation ofmRNA, stop codons, *etc.*)*.* The term "control element" as used herein includes, at a minimum, one or more components whose presence can influence expression; the term "expression control element" as used herein refers to one or more nucleic acid sequences that regulates the expression of a nucleic acid sequence to which it is operably linked. An expression control element operably linked to a nucleic acid sequence controls transcription and, as appropriate, translation of the nucleic acid sequence. Thus an expression control element can include, as appropriate, promoters, enhancers, transcription terminators, and/or a start codon (e.g., ATG) in front of a protein-encoding gene. Vectors can also include additional components such as, for example, leader sequences and fusion protein sequences. "Operably linked" refers to a juxtaposition wherein components are in a relationship permitting them to function in their intended manner.

By "promoter" is meant at least a minimal sequence that is sufficient to direct transcription. Promoters for use in or with the invention can be constitutive or inducible, as appropriate (see, e.g. Bitter et al. (1987) Methods in Enzymology 153: 516-544). Inducible promoters are activated by external signals or agents. Other promoter elements can include those which are sufficient to provide control of promoter-dependent gene expression for specific cell-types, tissues or physiological conditions; such elements may be located in the 5', 3', or intronic regions of the gene. Useful promoters also include "conditional promoters," which are active only under certain conditions. For example, a conditional promoter may be inactive or repressed when a particular agent is present (*e.g.*, a chemical compound), but may be active or derepressed when the agent is no longer present.

As used herein, "to transfect" or "transfection" refers to the introduction of one or more exogenous nucleic acids or polynucleotides into a eukaryotic cell. Transfection includes introduction in such a manner that a protein encoded by the nucleic acid or polynucleotide can be expressed. Transfection methods are known in the art and include a variety of techniques such as electroporation, methods using protein-based, lipid-based, and cationic-ion-based nucleic acid delivery complexes, viral vectors, "gene gun" delivery, and various other techniques.

A polynucleotide introduced into a cell (e.g., a DC) does not genetically modify the cell and is not stably maintained. The term "genetically modified" or "transformed" means containing and/or expressing a foreign gene or nucleic acid sequence which in turn modifies the genotype of the cell or its progeny; the phenotype of the cell is also altered. "Genetically modified" or "transformed" also refers to any addition, deletion, or disruption to a cell's endogenous nucleotides. Stable maintenance of an introduced polynucleotide typically requires that the polynucleotide either contains an origin of replication compatible with the host cell or integrates into a replicon of the cell such as an extrachromosomal replicon (e.g., a plasmid) or a nuclear or mitochondrial chromosome.

The term "transiently transfected" refers to a cell that has been transfected but which is not genetically modified and so progeny of the cell do not inherit the transformed genetic material (e.g., nucleic acid or polynucleotide). The genetic material may be RNA or it may be transcribed into RNA, and a protein encoded by the genetic material may be expressed. Such expression is referred to herein as "transient expression" Normally, transient expression is accomplished by not incorporating the transfected genetic material into the chromosome.

Dendritic cells are transiently transfected using RNA electroporation. Methods of RNA electroporation are well-known in the art. Generally, mRNA does not become a permanent part of the genome of the cell, either chromosomal or extrachromosomal. Any other methods that could be used to transiently express a desired protein are also contemplated within the scope of the invention. The methods do not involve permanent alteration of the genome (*i*.*e*., do not result in heritable genetic change to the cell) and thus avoid the disadvantages associated with the use of viruses, such as, for example, retroviruses and adenoviruses.

If desired, transformation of a cell with DNA may be carried out by conventional techniques known to those skilled in the art. For example, when the cell is a eukaryote, methods of DNA transformation include, for example, calcium phosphate co-precipitates, conventional mechanical procedures such as microinjection, electroporation, insertion of a plasmid encased in liposomes, and viral vectors. Eukaryotic cells also can be cotransformed with DNA sequences encoding a nucleic acid of interest and/or a second foreign DNA molecule encoding a selectable phenotype, such as those described herein. Another method is to use a eukaryotic viral vector, such as simian virus 40 (SV40) or bovine papilloma virus, to transiently infect or transform eukaryotic cells and express the protein. Following transformation, CD83 may be isolated and purified in accordance with conventional methods. For example, lysate prepared from an expression host (*e.g.*, bacteria) can be purified using HPLC, size-exclusion chromatography, gel electrophoresis, affinity chromatography, or other purification technique. Substantially pure proteins can also be obtained by chemical synthesis using a peptide synthesizer (*e.g.*, Applied Biosystems, Inc., Model 430A (ABI, Foster City, Calif., USA) or the like). CD83 may also be produced as described in the experimental section herein below.

The production of medicaments or pharmaceutical compositions comprising CD83 and at least one other immunosuppressive compound or at least one therapeutic composition used according to the invention and the use of such medicaments or pharmaceutical compositions occurs in the customary manner by means of common pharmaceutical technology methods and is thus readily provided by one of skill in the art. One of skill in the art will appreciate that different forms of administration will be suitable for different compounds and/or indications, and will be able to select the most appropriate method of administration. For example, psoriasis may be treated topically with a formulation suitable for administration to skin, while systemic lupus erythematosus may be treated by administration to the subject of a formulation suitable for intraperitoneal injection. Practitioners having skill in the art are familiar with criteria and methods for adjustment of dosages and administrations of compounds, such as, for example, assessment of results from conventional clinical and laboratory tests, including biochemical and immunological assays. Where appropriate, components of a medicament can be administered separately.

Generally, the compounds used according to the invention are processed together with suitable, pharmaceutically acceptable adjuvants and/or carriers to provide medicinal forms suitable for the various indications and types of routes of administration. A suitable pharmaceutical composition also can include stabilizers and preservatives. For examples of carriers, stabilizers and adjuvants, see, *e.g.,* Gennaro (1995) Remington's Pharmaceutical Sciences, 18th ed. (Mack Pub. Co., Easton, PA, U.S.). Accordingly, the medicaments can be produced in such a manner that the respective desired release rate is obtained, such as, for example a quick flooding effect or a sustained release effect.

Methods of treatment provided by the invention include the use of CD83 and at least one other immunosuppressive compound and, optionally, a therapeutic composition (*e*.*g*., an antigen) to induce tolerance and/or immunosuppression in a subject *in vivo.* Means of administering these substances to a subject include, but are not limited to, conventional and physiologically acceptable routes, such as, for example, oral, pulmonary, parenteral (*e.g.*, intramuscular, intra-articular, intraperitoneal, intravenous (IV) or subcutaneous injection), inhalation (via a fine powder formulation or a fine mist (aerosol)), transdermal, intradermal, nasal, vaginal, rectal, or sublingual routes of administration. These substances (*i.e*., the CD83, the at least one other immunosuppressive compound, and optionally the therapeutic composition) may be administered with a carrier, and they may be administered in the same formulation and via the same route of administration or they may be administered in different formulations and/or via different routes of administration. Preferably, each of the substances is administered at its optimal dosage so as to obtain optimal therapeutic effect of the coadministration. For example, administration of antibodies may be via intravenous injection, while administration of CD83 may be via intraperitoneal injection and administration of an immunosuppressive compound may be via oral dosing with a pill.

Carriers comprise any suitable physiological solution or dispersant or the like. The physiological solutions comprise any acceptable solution or dispersion media, such as saline or buffered saline. The carrier may also comprise antibacterial and antifungal agents, isotonic and adsorption delaying agents, and the like. Except insofar as any conventional media, carrier or agent is incompatible with the active ingredient, its use is contemplated. The carrier may further comprise one or more additional compounds, including but are not limited to cytokines such as, for example, interleukin-10 (IL-10) and TGF-β.

When the pharmaceutical composition comprises a nucleic acid for administration to a certain species of animal, the nucleic acid for use in the invention may be derived from that species. For example, when a pharmaceutical composition comprising a nucleic acid (*e.g*., DNA or RNA) encoding CD83 is to be administered to humans, the nucleic acid may comprise the sequence of the human form of CD83 protein. Nucleic acids for use in the invention can be administered in conjunction with agents that increase cell membrane permeability and/or cellular uptake of the nucleic acids. Examples of these agents are polyamines as described for example by Antony et al. (1999) Biochemistry 38: 10775-10784; branched polyamines as described for example by Escriou et al. (1998) Biochem. Biophys. Acta 1368: 276-288; polyaminolipids as described for example by Guy-Caffey et al. (1995) J. Biol. Chem. 270: 31391-31396; DOTMA as desribed by Feigner et al. (1987) Proc. Nat'l. Acad. Sci. USA 84: 7413-7417 and cationic porphyrins as described for example by Benimetskaya et al. (1998) Nucl. Acids Res. 26(23): 5310-5317.

Compositions, medicaments and methods of treatment provided by the invention also include tolerogenic DCs and regulatory T cells produced according to the methods described in the invention and the use thereof to induce tolerance in a subject *in vivo*. Means of administering the tolerogenic DCs and/or regulatory T cells to a subject include, but are not limited to, conventional and physiologically acceptable routes, such as, for example, intraperitoneal, intravenous (IV) or subcutaneous injection. The cells (*i.e*., the tolerogenic DCs and/or regulatory T cells produced by methods disclosed in the invention) may be administered with a carrier. Such carriers comprise any suitable physiological solution or dispersant or the like, such as, *e.g.,* saline or buffered saline. The carrier may also comprise antibacterial and antifungal agents, isotonic and adsorption delaying agents, and the like. Except insofar as any conventional media, carrier or agent is incompatible with the active ingredient (*i.e*., the tolerogenic DCs and/or regulatory T cells), its use in the compositions is contemplated.

As is familiar to those of skill in the art, dosage of the cells to be administered *in vivo* is determined with reference to various parameters, including the species of the host, the age, weight and disease status. Dosage also depends upon the location to be targeted within the host, *e.g.* the site of transplantation of tissue from a donor. For example, direct targeting to the site of inserted tissue may require different dosages than administration into the blood stream of a mammalian host. The dosage is preferably chosen so that administration causes an effective result, which can be measured by molecular assays or by monitoring a suitable symptom in the subject. Dosages may range from about at least 1 x 10⁴ cells to about at least 1 x 10⁹ cells per administration. The dosage ranges from about 5 x 10⁵ cells to about 5 x 10⁷ cells. To achieve maximal therapeutic effect, several doses may be required.

Where appropriate, prior administration of cells may also be used. Thus, for example, where cells are to be used for the prolongation of survival of transplanted tissue, administration of the cells into the transplant recipient may be conducted prior to transplantation, such as, for example, one week prior to transplantation. Administration may also be conducted at the time of the transplant and afterwards (*e*.*g*., for one to two weeks after the transplant, or for as long as necessary based on clinical evaluation) to ensure acceptance or lack of rejection of the transplanted tissue. Where appropriate, treatments may also comprise more than one individual treatment described herein; thus, for example, a course of treatment for a patient may include administration of sCD83 and at least one other immunosuppressive compound and may also include administration of tolerogenic DCs.

In the following, various aspects of the invention are more particularly described via examples. However, the invention is not limited merely to the embodiments illustrated by these examples but rather provides benefits as more fully described herein in the application as a whole.

### EXPERIMENTAL

### General Methods:

For general techniques, see Current Protocols in Immunology, eds. Coico et al. (Wiley, Hoboken, NJ).

**Western Blotting/ Coomassie Blue Staining:** A typical procedure for evaluating sCD83 was as follows. For each sample of sCD83, 1 microgram of sCD83 was prepared with running buffer and 2 microliters β-mercaptoethanol. The sample was heated for 10 minutes at 96 degrees and then run on an Invitrogen 4-12% polyacrylamide gel. The gel was either directly stained with Coomassie Blue or, if desired, it was transferred to a membrane. The membrane was probed with 1/1000 CD83 4B5 monoclonal primary antibody and then with 1/20,000 goat anti-Rat secondary antibody; signal was developed with Amersham chemiluminescence reagents.

**Primate Mixed Lymphocyte Reaction ("MLR"):** PBMCs were isolated from blood samples of two different cynomolgus monkeys using Ficoll gradient separation. The stimulator cells were rendered non-proliferative with Mitomycin C or radiation treatment. Stimulator cells and responder cells were seeded at 3 x 10⁵ cells/well and treated with titrating amounts of sCD83. At 96 hours, ³H-Thymidine (1 µCi/well) was added to the cultures. Cells were harvested 18 to 20 hours later and analyzed to determine the amount of T-cell proliferation.

**Staining for TNF-α, CD86 and CCR5:** Staining for TNF-α (intracellular) and CD86 and CCR5 (surface) were performed as follows. Whole blood samples were obtained from cynomologous monkeys and incubated with sCD83 for 12 hours. Samples were stimulated with 100 ng/mL LPS and 100 U/mL IFN-γ for 6 hours. Intracellular staining for TNF-α was performed using a commercially available Fix/Perm kit. Alternately, surface staining for CD86 and CCR5 was performed using standard techniques. After staining, red blood cells were lysed and results were analyzed with a BD Flow Cytometer (Becton Dickinson).

**B cell Proliferation Protocol:** Single cell suspensions of mouse spleen cells were made, and red blood cells were lysed using ACK lysing buffer (see Current Protocols in Immunology, eds. Coico et al. (Wiley, Hoboken, NJ)). B-cells were purified using CD19 MACS beads, and pure B-cells at 10⁵ cells/ per well were pre-incubated for three hours with titrating amounts of sCD83. B-cells were then stimulated with 2 µg/mL LPS. At 48 hours, 1 µCi of ³H-thymidine/well was added to the culture. Cells were harvested 18 to 20 hours later to determine the amount of B-cell proliferation.

**Antibody Production Protocols for *in vitro* Work:** IgM and IgG production was as follows. A pure population of 2x10⁶ B-cells/well (from C57B1/6 mice) were cultured with 20µg/mL of LPS (to induce antibody production) in the presence of a titrating concentration of sCD83 (15 to 90 µg/mL). Supernatants were harvested after 72 hours of culture, and the production of IgM and IgG in the supernatants was measured by ELISA.

Isotype Switching: Pure populations of 2 x 10⁶ B cells/well (from C57B1/6 mice) were cultured with one of the following treatments: 20µg/mL of LPS (to induce IgG2b); 20µg/mL LPS, 800 U/mL IL-4, and 150 U/mL IL-5 (to induce IgG1); or 20µg/mL LPS & 1000 U/mL IFN-γ (to induce IgG2a). For each treatment, some cells were also cultured with 60 µg/mL sCD83 and some were designated as No Treatment controls. Cells were harvested after 72 hours, and given a brief acid wash to remove Fc receptor bound immunoglobulin. Cells were then stained with one of the following, to detect surface IgG2b, IgG1 and IgG2a respectively: anti-IgM-FITC & anti-IgG2b-RPE; anti-IgM-FITC & anti-IgG1-RPE; or anti-IgM-FITC & anti-IgG2a-RPE.

**Antibody Production Protocols for *ex vivo* Work:** B cell proliferation: C57B1/6 mice were given a heterotopic cardiac allograft from a C3H mouse. Mice were then left untreated or were treated with a daily dose of 100 µg of sCD83 by intraperitoneal injection. Mice were then sacrificed on day 8, which was approximately the mean survival time of untreated mice. B-cells were then harvested from the mouse spleens and pure populations of 10⁵ B-cells/well were cultured with 2µg/mL of LPS to stimulate the cells (with no added sCD83). After 48 hours of culture, ³H-thymidine was added, and after an additional 18-20 hours of culture, the cells were harvested and ³H-thymidine incorporation was measured.

IgM & IgG production: C57B1/6 mice were given a heterotopic cardiac allograft from a C3H mouse. Mice then were left untreated or were treated with a daily dose of 100 µg of sCD83 by intraperitoneal injection. Mice were then sacrificed on day 8, which was approximately the mean survival time of untreated mice. B cells were harvested from the spleens of recipient mice and cultured at 2 x 10⁶ B-cells/well with 20ug/mL LPS (to induce antibody production, with no added sCD83). After 72 hours of culture, supernatants were harvested and IgM and IgG production measured by ELISA.

**Transplantation:** Procedures and criteria for selection of mouse strains are known in the art. See, e.g., Wang et al. (2003) J. Immunol. 171: 3823-3836.

### Example 1: Production of Recombinant Soluble CD83

**Bacterial strain and plasmid:** The plasmid of pGEX2ThCD83ext containing the cDNA encoding the extracellular domain of CD83 (sCD83) fused with GST was used as the expression vector for the production of recombinant GST-hCD83ext, which was under the regulation of an IPTG-inducible *tac* promoter (see, *e.g*., Lechmann et al. (2002) Protein Expression and Purification 24: 445-452). Due to the design of a thrombin cleavage site at the junction between GST and hCD83ext, the final sCD83 product has four extra amino acids (Gly-Ser-Pro-Gly) at the amino terminal.

**Cultivation of GST-sCD83:** The *E*. *coli* production strain, kindly provided by Dr. Alexander Steinkasserer, was stored frozen at -80°C, was revived by streaking it onto an LB agar plate (5 g/L NaCl, 5 g/L Bacto yeast extract, 10 g/L Bacto tryptone, and 15 g/L Bacto agar) containing 50 µg/mL ampicillin (Amp). The plate was incubated at 37 °C for approximately 15 hours. A few isolated single colonies were used to inoculate 600-mL LB medium containing 50 µg/mL Amp. The culture was then incubated at 37°C on a rotary shaker at 200 rpm for approximately 16 hours. The seed culture (of up to 600 mL) was used to inoculate a bioreactor (MBR Bioreactor AG, Wetzikon, Switzerland) containing 1 to 15 liters working volume of culture medium (5 g/L NaCl, 20 g/L Bacto yeast extract, 20 g/L Bacto tryptone, and 5 g/L glucose). When cells grew to a designated cell density of 2.0 ± 0.2 OD₆₀₀, the culture was supplemented with 0.5 mM isopropyl β-D-thiogalactopyranoside (IPTG) for induction of GST-hCD83ext production. Antifoam 289 (Sigma, St. Louis, MO, USA) at 50 µL/L was also added to the culture to avoid excessive foaming during cultivation. For aeration, the bioreactor was purged with filter-sterilized air at 1.5 vvm. The culture pH was kept at 7.00 ± 0.15 by adding 3 N NH₄OH or 3 N H₃PO₄ using a combination of a pH electrode (Ingold Messtechnik AG, Zurich, Switzerland), a pH controller (Model pH-40, New Brunswick Scientific Co., Edison, NJ, USA), and two peristaltic pumps (Watson Marlow, Falmouth, UK). After induction, the bioreactor was operated at 28 °C and 600∼650 rpm for 6 hours.

**Downstream processing of sCD83:** After cultivation, cells were harvested by centrifugation at 6000×g and 2°C, weighed, and stored at -80 °C for later use. Typically, cell paste at approximately 20 g wet cell weight (wcw) could be obtained from 1 liter of culture having a cell density of 8∼10 OD₆₀₀, implying that 1 g wcw was approximately equivalent to 400∼500 OD₆₀₀-units. To prepare the lysate for purification of hCD83ext, a cell suspension at 0.05 g-wcw/mL was prepared by suspending an appropriate amount of frozen cell paste in phosphate-buffered saline (PBS, pH 7.3). A batch of 100-mL cell suspension at approximately 20∼25 OD₆₀₀ was sonicated intermittently (0.5 seconds on/0.5 seconds off) for 4 minutes using an ultrasonic processor with a regular tip (Misonix, Farmingdale, NY, USA). The processed cell lysate was then centrifuged at 15,000×g and 2°C for 15 minutes to remove cell debris. The supernatant containing total soluble proteins was filtered with a 0.45-µm filter before subsequent chromatographic treatment for protein purification.

To capture the GST-CD83 fusion protein with GST affinity chromatography, 100 mL of the above-prepared lysate was loaded into a 20-mL GSTrap column (GE Healthcare, Baie d'Urfé, Québec, Canada) at a flow rate of 4 mL/min. While binding to the column, the GST-hCD83ext fusion was cleaved *in situ* with 10 U/mL thrombin (*i*.*e*., "on-column cleavage"). To do this, the PBS binding buffer in the GSTrap column bound with GST-hCD83ext fusion was replaced by the same volume of the binding buffer containing 10 U/mL thrombin through manual injection into the column. The cleavage was conducted at ambient temperature for approximately 2.5 h and the bulk liquid in the GSTrap column, primarily containing hCD83ext contaminated with thrombin, was ejected using 1 column volume of binding buffer. The GST moiety along with the rest of undigested GST-hCD83 was then eluted using the elution buffer. To maintain a good performance for the on-column cleavage, the GSTrap column was cleaned with 1M NaOH and 6M guanidine HCl to remove various protein contaminants trapped within the column.

A polishing step was performed to remove thrombin and other contaminant proteins from CD83. An FPLC system (AKTApurifier UPC 10, GE Healthcare) equipped with a strong anion-exchange column (30-mL HiTrap Q HP in XK16/20, GE Healthcare) was used for the processing, although an HPLC system can also be used to perform this purification step. The loading and elution buffers used were Tris buffer (20 mM, pH 7.5) with 50 mM NaCl and Tris buffer with 1 M NaCl, respectively. First, the anion-exchange column of the FPLC system was equilibrated with the loading buffer. Then, the protein solution eluted from the GSTrap column as described above was loaded into the column. The fractions containing hCD83ext were eluted, pooled, and concentrated with ultrafiltration using a high-pressurized, stirred cell (Amicon, Model 8050 with YM10 disk, Millipore Canada, Cambridge, Ontario, Canada) or similar ultrafiltration unit, and then stored at -20 °C. This product accumulation was conducted on a daily basis until the accumulated amount became large enough for processing for further endotoxin removal.

For further endotoxin removal, approximately 1 g of the accumulated sCD83 was treated with anion-exchange chromatography using the same experimental set-up and protocol for the polishing step. All the buffers for this processing step were prepared using water for injection (WFI). The fractions containing hCD83ext were pooled and concentrated with ultrafiltration using a high-pressurized stirred cell. Finally, the CD83 product fractions can be further subjected to filtration using with the Mustang E filter (Pall Life Sciences, Mississauga, Ontario, Canada) or similar filtration unit for endotoxin removal and then filter-sterilized. The endotoxin of each product fraction was assayed.

**Analytical protocols:** The culture sample was appropriately diluted with saline solution for measuring cell density in OD₆₀₀ with a spectrophotometer (DU^{®}520, Beckman Coulter, Fullerton, CA, USA). For the preparation of cell extract for analysis, cells in the amount of 40 OD₆₀₀-units (defined as 'OD₆₀₀×mL') were centrifuged at 6000×*g* at 2°C for 10 min. The cell pellet was resuspended in 3 mL PBS, disrupted by French Press (Thermo Electron Corporation, USA), and then centrifuged at 15,000×*g* at 2°C for 15 minutes to remove cell debris. Cell disruption may also be induced using other physical (e.g., sonication) or chemical methods. The supernatant containing soluble proteins was used for GST assay and SDS gel. The pellet containing insoluble proteins and cell debris was washed with phosphate buffer, resuspended in TE/SDS buffer (10 mM Tris HCl, pH 8.0, 1 mM EDTA, 1% SDS), and heated to 100°C for 5 minutes to allow dissolution. The protein content of the pellet was analyzed as the insoluble fraction.

GST function was assayed at ambient temperature using substrates 1-chloro-2,4 dinitrobenzene (CDNB) and glutathione. GST (and its fusion) catalyzes a reaction between the two substrates to form a conjugate product that can be detected at 340 nm. One unit (U_{GST}) is defined as the amount of enzyme that causes an increase in absorbance at 1 OD₃₄₀/min. The volumetric activity (in U_{GST}/mL) is the product of the specific activity (in U_{GST}/OD₆₀₀-unit) and cell density (in OD₆₀₀). Endotoxin was assayed using a commercial assay kit (LAL QCL-1000, Cambrex Bio Science Inc., Walkersville, MD, USA); the volumetric endotoxin concentration (in EU/mL) is the product of the specific endotoxin concentration (in EU/mg) and protein concentration (in mg/mL). Sodium dodecyl sulfate-polyacrylamide gel electrophoresis (SDS-PAGE) was performed in a Mini-PROTEAN^{®}II electrophoresis cell (Bio-Rad, Hercules, CA) using a 12.5% polyacrylamide separating gel stacked by a 4% polyacrylamide stacking gel. Protein samples of the cell extract (both soluble and insoluble fractions at 0.12 OD₆₀₀-unit) and the purified products at appropriate amounts were loaded. Electrophoresis was conducted at a constant voltage of 200 V for approximately 45 min. The gel was stained with Coomassie blue or silver nitrate, dried, and scanned to confirm the removal of endotoxin from the processed sample. Purified protein was frozen and stored in PBS (phosphate-buffered saline) (pH 7.5) at either - 20°C or - 80°C.

### Example 2: Soluble CD83 Does Not Induce Global Immunosuppression in a Mouse Model of Autoimmune Disease

Experimental Autoimmune Encephalomyelitis ("EAE") serves as an animal model of Multiple Sclerosis, a disease that shares some pathologic characteristics with Systemic Lupus Erythematosus ("SLE"), including dense autoaggressive CD4⁺ lymphocytic infilrates of affected tissues. It has been reported that soluble CD83 can reduce paralysis resulting from the induction of EAE (see, e.g., Zinser et al. (2004) J. Exp. Med. 200: 345-351). We wished to determine whether the immunosuppression induced by CD83 in the context of this mouse disease model was specific or whether it was a global immunosuppression.

In order to induce EAE, mice were immunized with a peptide derived from myelin oligodendrocyte glycoprotein (MOG), a constituent of the myelin sheath surrounding neurons of the central nervous system, and with pertussis toxin (see, *e.g.,* Zinser et al. (2004) J. Exp. Med. 200: 345-351). Beginning ten days later, the mice were inoculated with soluble CD83 (100µg) every day for ten days. 28 days after the last injection of soluble CD83, mice were injected subcutaneously with keyhole limpet hemocyanin ("KLH," 10 µg/mouse). Ten days later, spleen cells were removed from the mice and restimulated with different doses of KLH. Lymphocyte proliferation in response to this stimulation was measured by [³H]-thymidine incorporation. Results (shown in Figure 1) demonstrated that lymphocytes from these mice proliferated in response to KLH, indicating that the animals were immunocompetent and that soluble CD83 did not induce long-lasting, global immunosuppression.

### Example 3: Characterization of the Endpoint of the CD83 Window of Effectiveness

Mice were evaluated to determine the period of time after administration of CD83 in which a subject would be tolerized to another administered antigen. Soluble CD83 (100 µg) was administered to CBA mice by injection 10 times every second day. After 11 days, treated animals were immunized with keyhole limpet hemocyanin (KLH, 10 µg). Ten days later, spleen cells were removed and re-stimulation assays were performed as described in International patent publication WO 2004/046182, the contents of which are incorporated by reference. Results (shown in Figure 2) demonstrate that at this immunization interval, the subject is not tolerized to the antigen.

Another similar experiment was performed in which spleen cells were removed from the treated animals after 22 days and evaluated by re-stimulation assay; these results were similar to those described above and are shown in Figure 3.

### Example 4: Production of Tolerogenic DCs from Murine Bone Marrow Precursor Cells

C57/BL6 mice and BALB/C mice (male or female; Charles River, Wiga, Sulzfeld, Germany) were used at the ages of between 1 and 4 months. Generation of bone-marrow-derived dendritic cells ("BM-DCs") was performed as described in Lutz et al. (1999) J. Immunol. Meth. 223: 77. RPMI 1640 (Life Technologies, Karlsruhe, Germany) was supplemented with 100 U/ml penicillin (Sigma), 100 µg/ml streptomycin (Sigma), 2 mM L-glutamine (Sigma), 50 µg/ml ME (Sigma), and 10% heat-inactivated filtered FCS (PAA, Colbe, Germany). GM-CSF was used at 200 U/ml (PrepoTech/Tebu, Rocky Hill, N.J.) on days 0, 3, 6 and 8 of the incubation period.

An allogenic MLR assay was performed as follows: CD4⁺ and CD8⁺ T cells were isolated from inguinal and mesenchymal lymph nodes of BALB/C mice and co-cultured (2 x 10⁵ cells/well) for 3 days with BM-DCs (at different ratios) that had been processed through day 9 of the preparation protocol for BM-DCs. Culture was in 96-well culture dishes, and the media was 200 microliters RPMI 1640 supplemented with 100 U/ml penicillin, 100 pg/ml streptomycin, 2 mM L-glutamine, 50 µg/ml ME, and 10% heat-inactivated filtered FCS. Cells were pulsed with [³H]-thymidine (1 µCi/well; Amersham Pharmacia Biotech) for 16 hours. The culture supernatants were harvested onto glass fiber filters using an Inotech 1H-110 harvester (Inotech, Dottikon, Switzerland), and filters were counted in a Wallac 1450 microplate counter (Wallac, Turku, Finnland).

Results of the allogenic MLR assay showed that cluster formation between mouse immunostimulatory dendritic cells and mouse T cells was inhibited by soluble CD83 (here, hCD83ext). In addition, soluble CD83 inhibited in a concentration-dependent manner the ability of murine immunostimulatory dendritic cells to stimulate T cells, so that the T cells did not proliferate in response to exposure to the DCs.

### Example 5: sCD83 Suppresses B-Cell Proliferation and Function Ex Vivo

The effect of sCD83 on B-cells was measured in a series of experiments, as follows.

Effect of sCD3 on proliferation of transplant recipient B-cells ex *vivo*: Hearts of C3H mice were transplanted into the peritoneal cavity of C57BL/6 mice. Eight days after transplantation (when the transplant would typically be rejected in untreated mice), the mice were sacrificed. B cells were purified from recipient mice, cultured, and stimulated with LPS. ³H-thymidine incorporation was measured as counts per minute (cpm). Results are shown in Figure 4 and demonstrate that sCD83 suppresses *ex vivo* B-cell proliferation.

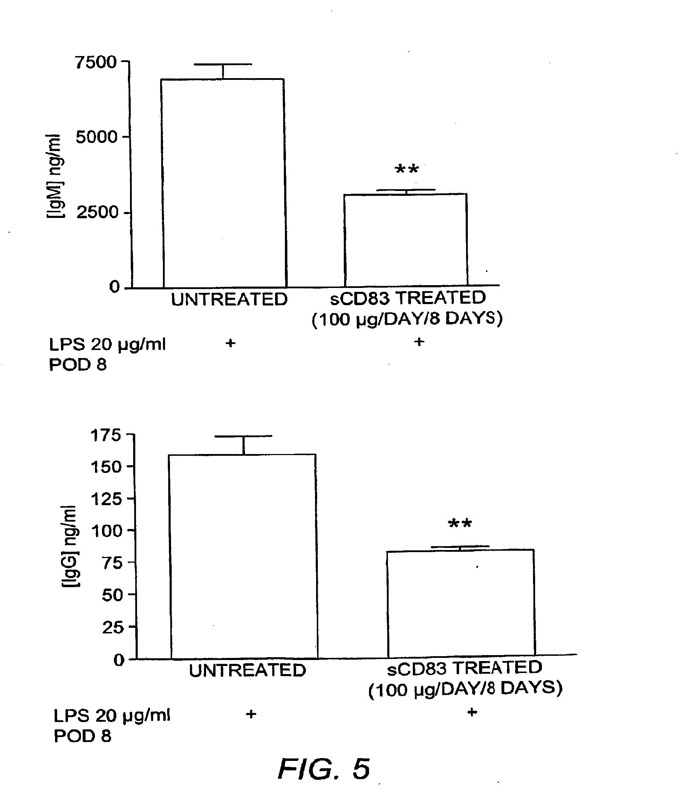

Effect of sCD83 on *ex vivo* antibody production by B cells: Hearts of C3H mice were transplanted into the peritoneal cavity of C57BL/6 mice. Eight days after transplantation (when the transplant would typically be rejected in untreated mice), the mice were sacrificed. B cells were purified from recipient mice, cultured, and stimulated with LPS. After 72 hours of culture, supernatants were collected and IgM and IgG production was measured using ELISA. Results are shown in Figure 5 and demonstrate that sCD83 suppresses *ex vivo* antibody production.

### Example 6: sCD83 Coadministered with Cyclosporine, Tacrolimus, or MMF Increases Cardiac Graft Survival in Allograft Transplant Recipients

Hearts were removed from donor mice as indicated and transplanted into the peritoneal cavity of recipient mice (*i.e*., intra-abdominal, heterotopic heart transplantation). Recipient mice were treated as indicated, with some treatments beginning prior to transplantation. Mean survival time of the transplanted tissue was determined for each treatment.

C57BL/6 donor mice and BALB/c recipient mice were used. Treatments and survival of the transplanted tissue for each treatment group are as shown in Table 1. In this set of experiments, treatment with sCD83 began with a daily intraperitoneal injection of 100 µg of sCD83 from "day -1" (*i.e*., the day before transplantation) through day 7 and then continued with injections every other day through day 28. In this application, "day -1" generally refers to the day before transplantation, which referred to as "day 0." Treatment with cyclosporine was by subcutaneous injection daily either with a subtherapeutic dose (5 mg/kg/day) or a high dose (15 mg/kg/day). Treatment with tacrolimus was by daily oral administration of a subtherapeutic dose of 8 mg/kg/day, and treatment with MMF was by daily oral administration of a low dose of 80 mg/kg/day. The results demonstrate that sCD83 coadministered with cyclosporine, tacrolimus, or MMF increased cardiac graft survival in allograft transplant recipients.

**Table 1: sCD83 Coadministered with Cyclosporine, Tacrolimus, or MMF Increases Heart Graft Survival of C57BL/6 Hearts in BALB/c Recipients**

| C57BL/6 to BALB/c | Individual Survival (days) | MST (days) |
|---|---|---|
| Untreated | 8, 8, 9, 9 | 8.5 ± 0.6 |
| sCD83 | 15, 15, 16, 17 | 15.8 ± 1.0 |
| CsA (5 mg/kg, historical) | 9, 10, 10, 10, 10, 11, 11 | 10.1 ± 0.3 |
| sCD83 + CsA (5 mg/kg) | 18, 21, 23, 25 | 21.8 ± 3.0 |
| CsA (15 mg/kg, historical) | 14, 15, 15, 16, 16, 17 | 15.5 ± 1.1 |
| sCD83 + CsA (15 mg/kg) | 28, 35, 40, 41, 42 | 37.2 ± 5.8 |
| Tacrolimus | 13, 14, 14, 15, 16, 18, 19, 21 | 16.3 ± 1.0 |
| sCD83 + Tacrolimus | 20, 27, 28, 30, 32, 35 | 28.7 ± 5.1 |
| sCD83 + MMF | 20, 22, 23, 26, 27, 28 | 24.3 ± 3.1 |

In further experiments, transplant recipients were treated with a shorter course of administrations and a different route of administration. Mice were generally treated as described above; hearts were removed from C57BL/6 donor mice and transplanted into the peritoneal cavity of BALB/c recipient mice. Soluble CD83 at a dose of 100 µg per mouse per day and cyclosporine at a dose of 15 mg per kg per day were administered intravenously to recipient mice from day -1 to day 7. Mean survival time of the transplanted tissue was over 100 days.

### Example 7: sCD83 Inhibits Allo-Antibody Induction in Transplant Recipients

The transplantation of tissues between non-histocompatible individuals stimulates transplant *r*ejection. The rejection process can involve both the cellular and humoral arms of the immune response. Acute rejection of a transplanted organ can be mediated by an antibody response directed against donor-specific allo-antigens (*i.*e., foreign histocompatibility antigens). Alternatively, allo-antibody responses can be induced as a consequence due to donor tissue destruction facilitated by a cell mediated response. In either case, the presence of an allo-antibody response in the recipient can be detected by measuring the titer of antibodies in recipient sera that can bind to donor-derived cells, typically by staining donor-derived splenocytes. Because sCD83 increased survival time of grafts in transplantation experiments, we wished to determine whether sCD83 affected the induction of alloantibody in transplant recipients.

In these experiments, sera were collected from transplant recipients (from control-treated, sCD83-treated and treatment-naïve individuals). Splenocytes were removed from the transplant donor(s) and incubated with the transplant recipient sera. The binding of antibodies from the allo-antisera to the surface of the transplant donor splenocytes was monitored selectively using antibodies specific for the Fc-fragments of mouse IgM and IgG. An advantage of this procedure is that it does not measure murine antibody expressed passively from the donor cells (*e.g*., B-cells within the donor splenocyte population) because the Fc-fragment is only accessible if the Ig is derived from an extra-cellular source (*i.e*., the recipient sera); the Fc-fragments of donor-derived Ig on the surface of donor B-cells, *etc*. will be embedded in Fc-receptors or retained intra-cellularly and thus will be unavailable for staining. Stained cells can be analyzed using FACS.

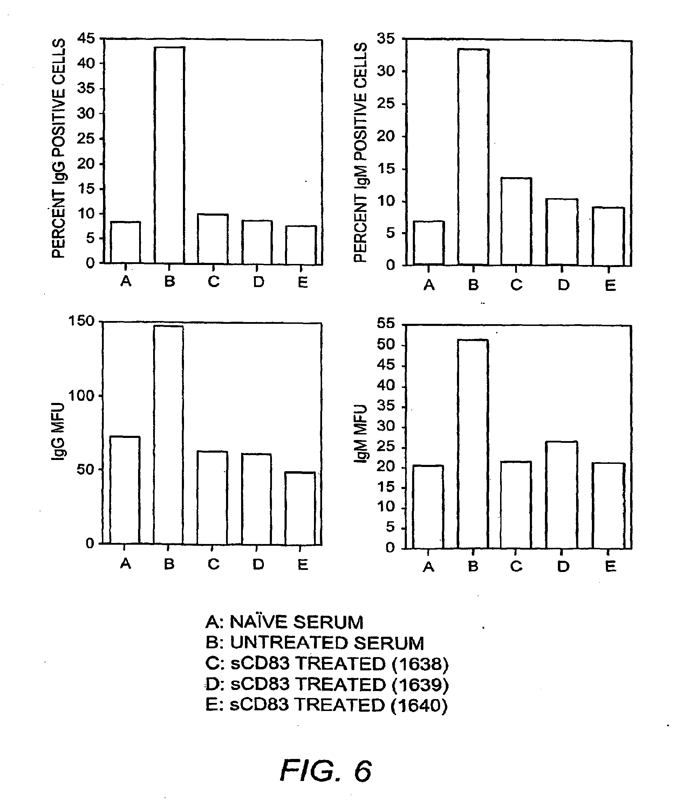

In the transplantation procedure, hearts were removed from C3H mice and transplanted into C57BL/6 mice, which were then either treated with sCD83 or left untreated. Sera were collected from the C57BL/6 transplant recipients eight days after transplantation (the approximate time when untreated mice would typically reject the transplant (*i.e*., show full tissue rejection)). This recipient sera was then used to stain splenocytes from the C3H donors, and allo-antibody binding was detected with FITC-conjugated antibodies specific to either the anti-IgG or anti-IgM Fc-fragment. The results showed that transplant recipients treated with sCD83 had low allo-antibody activity (i.e. low donor-specific binding of IgM or IgG) that was equivalent to levels in serum from naive mice (*i.e*., mice which had not received a transplant) (see Figure 6). C57BL/6 mice which received C3H heart transplants and were also treated with sCD83 had low allo-antibody activity, equivalent to serum derived from non-transplanted naive C57BL/6 mice. In contrast, transplant recipients that were untreated and so rejected the transplanted tissue had detectable allo-antibody that was present at significantly higher levels than in the control. Sera from untreated transplant recipients was also able to stain C3H-donor-derived splenocytes above control levels. These experiments demonstrate that sCD83 suppresses donor-specific antibody production by B cells against foreign antigens (e.g., transplanted tissue) and can prevent organ rejection.

### Example 8: sCD83 Coadministered with Sirolimus and CD45RB mAb Achieves Long-Term Cardiac Graft Survival in Allograft Transplant Recipients

Hearts were removed from donor mice as indicated and transplanted into the peritoneal cavity of recipient mice (*i.e*., heterotopic transplantation). Recipient mice were treated as indicated, with some treatments beginning prior to transplantation. Survival of the allograft was monitored and scored as follows: "A," beating strongly; "B," mild decline in the intensity of pulsation; "C," noticeable decline in the intensity of pulsation; "D," complete cessation of cardiac impulses. Mean survival time of the transplanted tissue was determined for each treatment.

In a first set of experiments, C3H donor mice and BALB/c recipient mice were used. Treatments and survival of the transplanted tissue for each treatment group are as shown in Table 2. In this set of experiments, sCD83 treatment began on day -1 (*i.e*., the day prior to transplantation) and lasted until day 28; each day, 100 µg of sCD83 was administered to each mouse by intraperitoneal injection. Sirolimus treatment began on day 0 (*i.e*., the day of transplantation) and continued until day 14; each day, 2 mg/kg of sirolimus was administered orally to each mouse. Anti-CD45RB treatment began on day 0 and continued until day 14; each day, 100 µg of anti-CD45RB (BioExpress, Inc., West Lebanon, New Hampshire) was administered to each mouse intravenously.

**Table 2: sCD83 Coadministered with Sirolimus and CD45RB mAb Achieves Long-term Heart Graft Survival of C3H Hearts in BALB/c Recipients**

| C3H-to-BALB/c | Individual Transplant Survival (days) | MST (days) |
|---|---|---|
| Untreated | 8, 8, 8, 9 | 8.3 ± 0.5 |
| Sirolimus | 12, 23, 25, 25 | 21.3 ± 6.2 |
| Anti-CD45RB mAb | 12, 15, 21 | 16.0 ± 4.6 |
| sCD83 | 9, 9, 10, 12 | 10.0 ± 1.4 |
| Anti-CD45RB mAB + sirolimus | 31, 33, 36 | 33.3 ± 2.5 |
| sCD83 + sirolimus | 52, 53, 62 | 55.7 ± 5.5 |
| sCD83 + anti-CD45RB mAb | 35, 37, 38 | 36.7 ± 1.5 |
| sCD83 + sirolimus + anti-CD45RB mAB | > 100 (x 4) | > 100 |

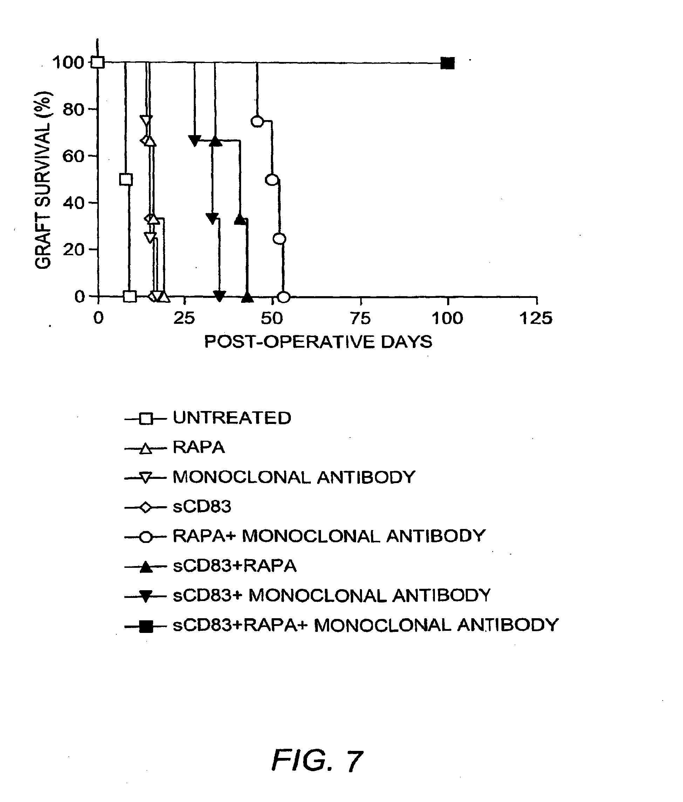

In a further set of experiments, C3H mouse hearts were transplanted into the peritoneal cavity of C57BL/6 mice (results shown in Table 3 and Figure 7). Without treatment, the heart grafts were rejected in 8.5 ± 0.6 days by acute cellular and humoral rejection, characterized by vasculitis, hemorrhage, thrombosis and infiltration of CD4⁺ CD8⁺ cells. High levels of IgG and IgM were also detected in the transplanted tissue as well as in the blood of the recipients. Treatment with sCD83 alone (*i.e*., intraperitoneal injection of 100 µg of sCD83 per mouse every day from days 1 through 7 and then every other day (*q.o.d*.) from days 8-16) reduced symptoms of acute rejection and increased heart graft survival to 10.7 ± 1.5 days; the same treatment with 400 µg of sCD83 doubled heart graft survival to 15 ± 1.0 days. Other treatments included subtherapeutic doses of either anti-CD45RB monoclonal antibody ("mAb") (*i.e*., 100 µg/mouse every day from days 0 through 14 by intravenous injection) or sirolimus (*i.e*., 2 mg/kg every day from days 0 through 14 by oral administration ("*p.o*.")). Subtherapeutic doses in these experiments were based on equivalent human doses.

Unexpectedly, sCD83 at the lower dose of 100 µg per injection showed a synergistic effect with these subtherapeutic doses of either anti-CD45RB mAb or sirolimus; the combined treatments with sCD83 further improved graft survival to 32 ± 3.6 days and 39.3 ± 4.7 days, respectively. The combined treatment of anti-CD45RB mAb and sirolimus increased survival to 50.3 ± 3.1 days. Remarkably, sCD83 in combination with both anti-CD45RB mAb and sirolimus effectively prevented acute rejection and induced graft tolerance with indefinite survival for more than 100 days (p<0.01 versus combined treatment with anti-CD45RB mAb and sirolimus). The transplanted hearts from mice treated with the combination of sCD83, anti-CD45RB mAb, and sirolimus *(i.e.,* "triple treatment") were examined on post-operative day 100 ("POD 100") and showed normal histology with no vasculopathy. This experiment demonstrated that treatment comprising coadministration of sCD83 with sirolimus and anti-CD45RB mAB induced long-term acceptance of transplanted heart tissue.

**Table 3: sCD83 in Combination with Sirolimus and Anti-CD45RB mAb Achieves Lons-term Heart Graft Survival of C3H Hearts in C57BL/6 Recipients**

| C3H-to-C57BL/6 | Individual Survival (days) | MST (days) |
|---|---|---|
| Untreated | 8, 8, 9, 9 | 8.5 ± 0.6 |
| Sirolimus | 15, 16, 19 | 16.7 ± 2.1 |
| Anti-CD45RB mAb | 14, 15, 15, 17 | 15.3 ± 1.3 |
| sCD83 (100 ug) | 9, 11, 12 | 10.7 ± 1.5 |
| sCD83 (266 ug) | 14, 15, 15 | 14.7 ± 0.6 |
| sCD83 (400 ug) | 14, 15, 16 | 15.0 ± 1.0 |
| Anti-CD45RB mAB + sirolimus | 46, 50, 52, 53 | 50.3 ± 3.1 |
| sCD83 + sirolimus | 34, 41, 43 | 39.3 ± 4.7 |
| sCD83 + anti-CD45RB mAb | 28, 33, 35 | 32.0 ± 3.6 |
| sCD83 + sirolimus + anti-CD45RB mAB | > 100 (x 4) | > 100 |

While the invention is not bound by any particular mechanism of operation, generally, transplantation of C3H tissue into BALB/c mice is considered to be a model system for antibody-mediated rejection, while transplantation of C3H tissue into C57BL/6 mice is considered to be a model system for T-cell mediated rejection. Therapy with sCD83 alone and coadministered with other substances improved graft survival in both systems, demonstrating that sCD83 can suppress undesired immune responses that are mediated by B-cells as well as those that are mediated by T-cells. In other words, sCD83 can suppress humoral graft rejection in a C3H-to-BALB/c mouse cardiac transplantation model and can also suppress acute cellular graft rejection in a C3H to C57BL/6 mouse cardiac transplantation model.

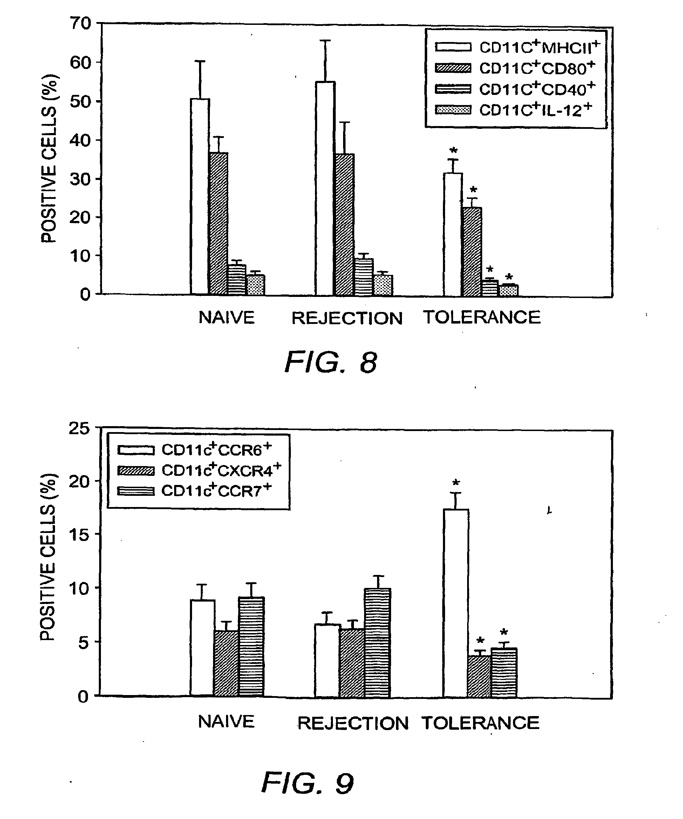

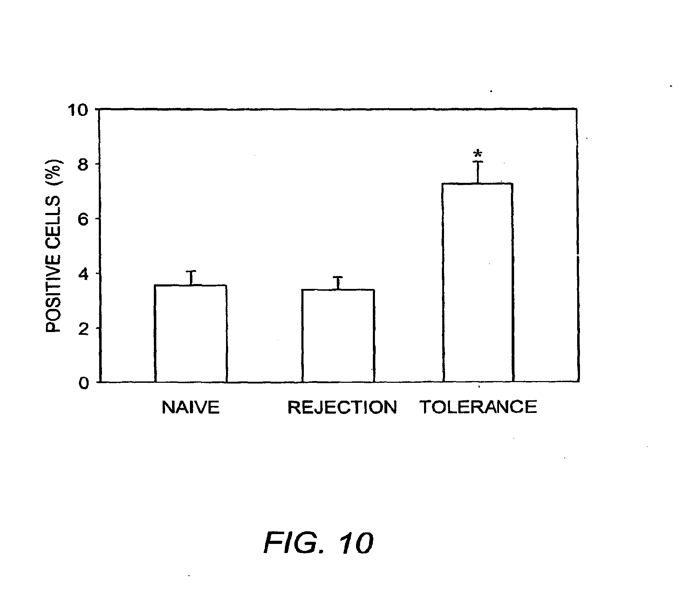

The recipient mice that received the triple-treatment of sCD83, anti-CD45RB mAb, and sirolimus were also examined to determine the effect of the treatment on generation of tolerogenic DCs *(i.e.,* CD11c⁺ MHCII^{low} CD40^{low} CD86^{low} IL-12⁻ cells) and regulatory T cells (CD4⁺ CD25⁺ Foxp3⁺ cells). An increased amount of both tolerogenic DCs and regulatory T cells was found in these mice when they were examined on post-operative day 100 (see flow cytometry results shown in Figure 8, Figure 9, and Figure 10). The CXCR6 marker is characteristic of immature DCs, while the CXCR4 and CCR7 markers are characteristic of mature DCs. Asterisks in Figures 9 and 10 indicate results that differed at p < 0.01. Thus, these results demonstrate that sCD83-based therapy induced the generation of both tolerogenic DCs and regulatory T cells.

### Example 9: sCD83 Suppresses In Vitro TNF-α Production and Surface Expression of mDC Activation Markers

We wished to examine the *in vitro* functional properties of human sCD83 on peripheral blood DCs, monocytes, and T cells. Mature DCs ("mDCs") from cynomolgus monkeys were identified using FACS with four-color staining as cells that were HLA-DR positive, lineage negative (*i.e*., CD3, CD20, CD14, and CD16 negative), and CD11c positive. Intracellular TNF-a, IL-6, and surface markers CD83, CD80, CD86, and CCR5 (which are increased in activated, mature DCs) were also monitored.

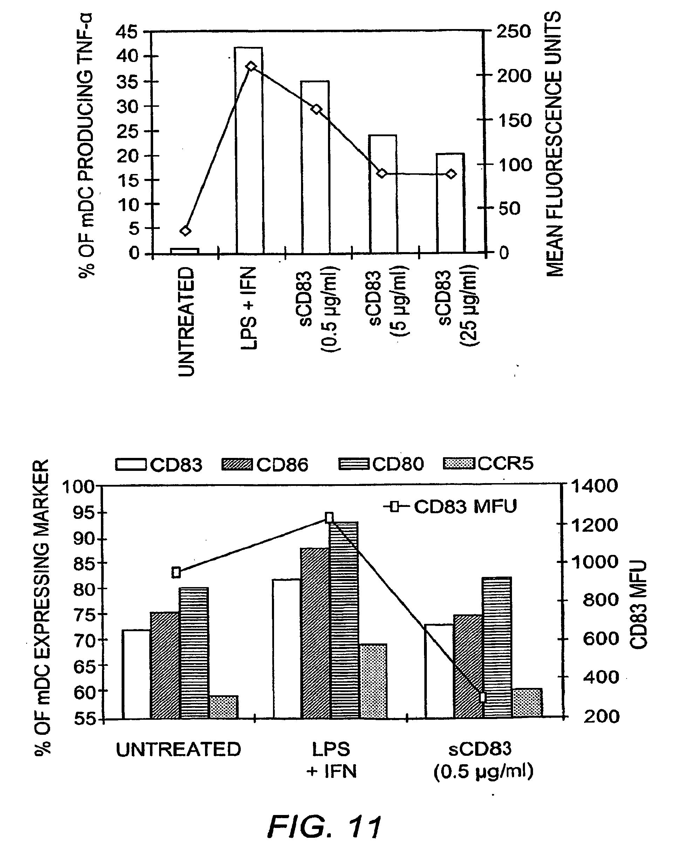

To evaluate the effect of sCD83 on mDCs, isolated PBMCs (1 x 10⁶ cells) from cynomolgus monkeys were incubated overnight with sCD83. Cells were stimulated with LPS (1 µg/ml) and IFN-γ (100 U/ml) for 6 hours. Intracellular TNF-α levels and surface markers were detected in mDCs using FACS. Results representative of one out of three experiments are shown in Figure 11; results were expressed as % positive cells and mean fluorescence units (MFU). The results show that sCD83 suppressed *in vitro* production of TNF-α by mDCs and also suppressed the surface expression of mDC activation markers CD83, CD86, CCR5, and CD80.

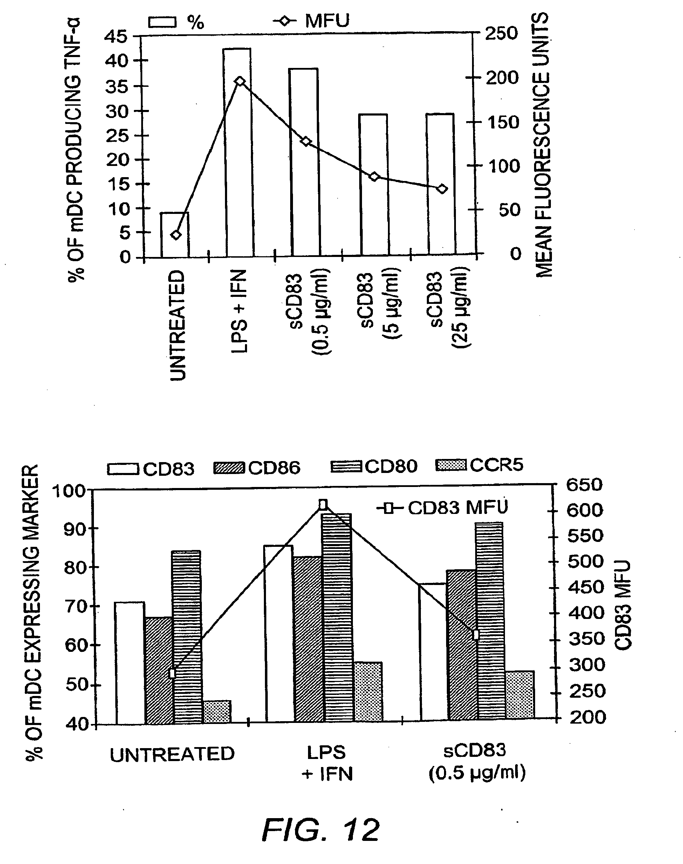

To evaluate the effect of sCD83 on monocytes, isolated PBMCs (1 x 10⁶ cells) from cynomolgus monkeys were incubated overnight with sCD83 (1 x 10⁶ cells). Intracellular TNF-α levels and surface markers were detected in monocytes using FACS. Results representative of one out of three experiments are shown in Figure 12; results were expressed as % positive cells and mean fluorescence units (MFU). The results show that sCD83 moderately suppressed the *in vitro* production of TNF-α by monocytes and also suppressed the surface expression of monocyte activation markers.

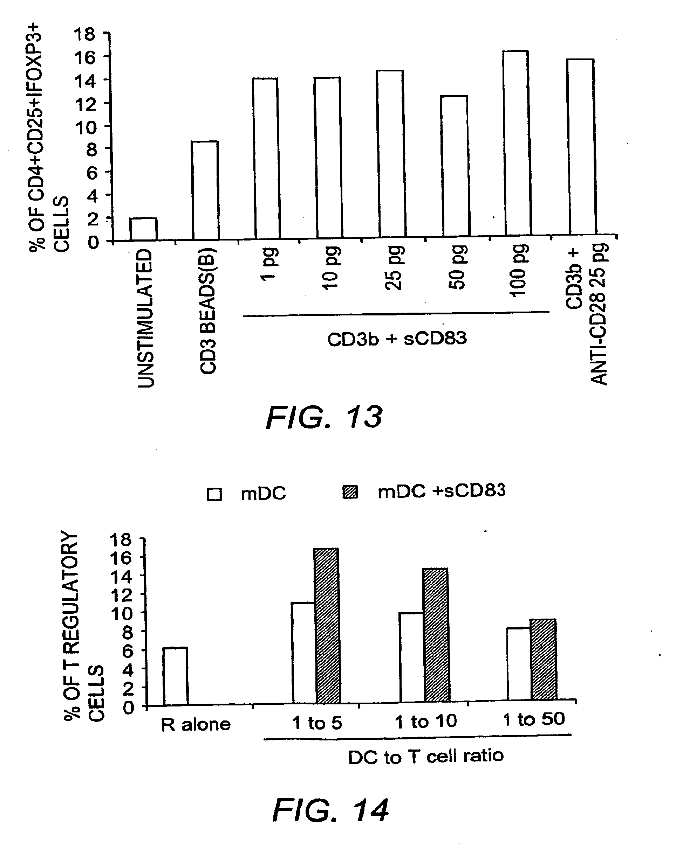

To evaluate the effect of sCD83 on CD4⁺ T cells, these cells were purified from cynomolgus monkeys and then stimulated in the presence or absence of sCD83 or anti-CD28 mAb for 6 days. Cells were then stained to identify regulatory T cells *(i.e.,* CD4⁺ CD25⁺ Foxp3⁺ cells) and analyzed by FACS. The results (shown in Figure 13) suggest that sCD83 promotes the generation of regulatory T cells.

Regulatory T cells were also prepared using dendritic cells prepared from cynomolgous monkeys, as follows. Cynomolgous monkey myeloid dendritic cells were generated from CD 14⁺ monocyte cells in the presence of GM-CSF and IL-4 for 7 days; soluble CD83 at 50 ug/ml was added to some cultures on days 3 through 7, and a maturation cocktail of IL-1β, IL-6, TNF-α and PGE-2 was added on days 5 through 7. T cells (1 x 10⁶/ml) were isolated by negative selection and stimulated at different ratios with the allogeneic mature dendritic cells that had been either treated or untreated with sCD83 (50 µg/ml). Cells were then stained to identify regulatory T cells (*i.e*., CD4⁺ CD25⁺ Foxp3⁺ cells) and analyzed by FACS. The results (shown in Figure 14) show that increased numbers of regulatory T cells were generated by dendritic cells treated with soluble CD83, particularly at the higher DC:T ratios tested.

### Example 10: sCD83 Suppression of B-Cell Proliferation and Function In Vitro

Evaluating effect of sCD83 on *in vitro* B-cell proliferation: C57BL/6 B-cells are stimulated with LPS in the presence or absence of sCD83. After 48 hours of culture, ³H-thymidine incorporation is measured as counts per minute (cpm). Results are evaluated to determine whether sCD83 suppresses *in vitro* B-cell proliferation.

Evaluating effect of sCD83 on IgM and IgG production by B cells: C57BL/6 B-cells are stimulated with LPS in the presence or absence of sCD83. After 72 hours of culture, supernatants are harvested and IgM or IgG production was determined using ELISA. Results are evaluated to determine whether sCD83 suppresses B-cell antibody production *in vitro.*

### Example 11: Production of Tolerogenic Dendritic Cells and Evaluation of Dendritic Cell Function

Tolerogenic dendritic cells can be produced as follows. Cells can be cultured using standard medium with 1% human plasma (*e.g*., RPMI 1640 (BioWhittaker, Verviers, Belgium) supplemented with glutamine (200 µg/ml, BioWhittaker, Verviers, Belgium), penicillin/streptomycin (20 µg/ml), 10 mM Hepes, pH 7.5 (Sigma-Aldrich), and 1% human plasma from a single donor (previously heat-inactivated by incubating at 56°C for 30 minutes). Human PBMCs can be isolated from buffy coats by sedimentation in Ficoll-Hypaque (Amersham Pharmacia Biotech, Freiburg, Germany), seeded onto 100 mm culture dishes coated with IgG (10 µg/ml γ-globulin from Cohn fraction; Sigma-Aldrich), and incubated at 37°C in 5% CO₂. After 1 hour and 7 hour incubations, non-adherent cell fractions are removed and the adherent cells further cultured in 1% human plasma medium supplemented with the cytokines GM-CSF (800 U/ml) and IL-4 (500 U/ml). On day 3 of the incubation period, fresh medium is added (including GM-CSF at 400 U/ml and IL-4 at 500 U/ml). On day 4 or 5, non-adherent cells are collected, counted, and transferred into new dishes at a density of 0.3-0.5 x 10⁵ cells/ml. For the final maturation step, 1% human plasma medium is supplemented with TNF-α (1.25 ng/ml), GM-CSF (40 U/ml), IL-4 (200 U/ml), prostaglandin E₂ (0.5 pg/ml; see, *e.g.,* Lechmann et al. (2001) J. Exp. Med. 194: 1813-1821), and soluble CD83 (4 µg/ml). Alternatively, for the final maturation step, the maturation cocktail comprises IL-1β, TNF-α, PGE₂, and soluble CD83 (4 µg/ml). On day 8, cells can be analyzed by FACS. Cells matured by this method reveal a clear reduction in the cell surface expression of CD80 *(e.g.,* from 96% to 66%) and CD83 (*e.g.*, from 96% to 30%) and an increase in CD14 positive cells when compared with normally matured DCs (see, *e.g.,* WO 2004/046182). Expression of other cell surface markers (*e.g.,* MHC Class I and II) may not be significantly affected. In other embodiments, cells can be matured according to the protocol as described above but soluble CD83 and the other immunosuppressive compound(s) can be added to the maturation cocktail after one, two, three, or more days (separately or together, as discussed above).

Dendritic cells may be evaluated using an allogenic mixed lymphocyte reaction ("MLR") assay, as follows. CD4⁺ and CD8⁺ T cells are isolated from buffy coats. Briefly, harvested non-adherent cell fractions are incubated with neuramidase-treated sheep erythrocytes, collected by Ficoll gradient centrifugation, and cultured in RPMI supplemented with 5% human serum from a single AB donor. Cells are then stimulated with different ratios of mature allogenic immunostimulatory DCs. Cells are incubated with different concentrations of soluble CD83 ("hCD83ext" as described in WO 2004/046182) or with BSA (BioRad) as a control, or are untreated. T cells (2 x 10⁵ cells/well) and DCs are co-cultivated for 4 days in RPMI supplemented with 5% human serum from a single AB donor in 96-well cell culture dishes. Cells are pulsed with [³H]-thymidine (1 µCi/well; Amersham Pharmacia Biotech) for 16 hours. The culture supernatants are harvested onto glass fiber filters using an IH-110 harvester (Inotech. Dottikon, Switzerland), and filters are counted in a Wallac 1450 microplate counter (Wallac, Turku, Finnland).

### Example 12: Determination of the Endpoint of the CD83 Window of Effectiveness in Response to Various Stimuli

Mice are evaluated essentially as described in Example 3 for their immune response following varying intervals between administration of CD83 and KLH1 *(i.e.,* 10 days, 8 days, 6 days, 4 days, 2 days, 1 day). An interval is defined for which restimulation using KLH does not provoke an immune response; this interval marks the end of the window of sensitivity in this experiment.

Subjects are evaluated essentially as described above, but other immune stimuli are examined. These protocols substitute third-party skin grafts for the KLH antigen. Other protocols substitute *Leishmania* infection during and after treatment with CD83 (on the day before CD83 treatment, the day after CD83 treatment, and three days after CD83 treatment). Other protocols substitute infection with LCMV (lymphocytic choriomeningitis virus) during and after treatment with CD83.

### Example 13: Evaluation of Memory T cell Reactivation in Response to CD83

Mice are infected with LCMV and 28 days later are injected with CD83. Peripheral Blood Mononuclear Cells (PBMCs) are evaluated for the presence of LCMV-specific Memory T cells that produce IFNγ and IL-2, but do not kill target cells.

### Example 14: Evaluation of Effect of CD83 on Regulatory T Cells

Antigen-specific T cells are produced *in vitro* and exposed to dendritic cells in the presence or absence of soluble CD83. The effect of CD83 is then evaluated by assaying T cells for proliferation (CFSE), apoptosis (Annexin V), cytokine production (IFN-γ and IL-10), and the presence of Foxp3; appropriate assays are known in the art.

T cells are isolated from mammals so that they can be cultured and/or assayed *in vitro.* PBMCs are separated from red blood cells and neutrophils using Ficoll-Hypaque density gradient centrifugation according to established procedures. Cells are washed with modified AIM-V (which consists of AIM-V (GIBCO) with 2 mM glutamine, 10 µg/ml gentamicin sulfate, and 50 µg/ml streptomycin) supplemented with 1% fetal bovine serum (FBS). T cells are enriched by negative or positive selection with appropriate monoclonal antibodies coupled to columns or magnetic beads according to standard techniques. An aliquot of cells is analyzed for cell surface phenotype including expression of CD4, CD8, CD3 and CD14.

In some instances, cells are washed and resuspended at a concentration of about 5 x 10⁵ cells per ml of AIM-V modified as above and containing 5% FBS and 100 U/ml recombinant IL-2 (rIL-2) (supplemented AIM-V). T cells can then be exposed to immunostimulatory or tolerogenic dendritic cells in the presence or absence of sCD83.

To stimulate proliferation, OKT3 monoclonal antibody (Ortho Diagnostics) can be added to a concentration of 10 ng/ml. The cells are plated in 24 well plates (0.5 ml of cell suspension per well) and are cultured at a temperature of about 37°C in a humidified incubator with 5% CO₂. Proliferation of T cells in response to a reactive composition such as OKT3 antibody can be monitored quantitatively by measuring, for example, ³H-thymidine uptake (see, *e.g*., Caruso et al. (1997) Cytometry 27: 71).

Analysis of the types and quantities of cytokines secreted by T cells during and after exposure to dendritic cells can be a measure of functional activity. Cytokines can be measured by ELISA or ELISPOT assays to determine the rate and total amount of cytokine production. See, *e.g.,* Fujihashi et al. (1993) J. Immunol. Meth. 160: 181; Tanquay and Killion (1994) Lymphokine Cytokine Res. 13: 259; Parkhurst et al. (1996) Immunol. 157: 2539.

### Example 15: Evaluation of Adoptive Transfer of Immunity using Regulatory T Cells Produced with CD83

Mice are immunized with 10 µg of KLH. CD83 (100 µg) is administered the day before, the day after, and three days after KLH immunization. Ten days after the KLH immunization, splenocytes are prepared from these animals and are transferred into naïve animals which are then immunized with KLH and assayed for an immune response to the antigen.

### Example 16: Adoptive Transfer of Tolerance using Tolerogenic DCs

Tolerogenic DCs prepared with a maturation step comprising soluble CD83 are prepared as described in Example 4. Immature DC are loaded with an antigen of interest, or a nucleic acid encoding an antigen of interest. These DC are transferred into a subject and the subject's immune response to the antigen is evaluated.

### Example 17: Coadministration of Soluble CD83 and a Transplanted Organ

Kidney transplants are performed on outbred, juvenile cynomolgus macaques; each macaque receives a kidney that was removed from another macaque. During the transplantation procedure, recipient macaques are injected intravenously or intraperitoneally with soluble CD83 at a dose of between 3 and 6 mg/kg/day. Administration is once per day or four times a day for 28 days. Control macaques receive the transplanted organ but no soluble CD83. Following the transplantation procedure, macaques are monitored for viability and blood supply of the transplanted organ.

## Claims

1. Soluble CD83 for use to prevent, cure, or alleviate at least one symptom of a disease or disorder caused by the dysfunction or undesired function of an immune response in combination with a first immunosuppressive compound, wherein said first immunosuppressive compound is selected from cyclosporine, tacrolimus, sirolimus, and anti-CD45RB mAb, wherein said disease or disorder is selected from the group consisting of an allergy; asthma; rejection of transplanted tissue; an immune response to a chronically administered substance; an autoimmune disease; and AIDS.

2. The sCD83 for use according to claim 1, wherein the sCD83 and the first immunosuppressive compound are for administration in the same formulation and via the same route of administration or are for administration in different formulations and/or via different routes of administration.

3. The sCD83 for use according to claim 1 or claim 2, wherein said first immunosuppressive compound is cyclosporine.

4. The sCD83 for use according to claim 1 or claim 2, which is for use in combination with a second immunosuppressive compound, wherein said first immunosuppressive compound is sirolimus and said second immunosuppressive compound is anti-CD45RB mAb.

5. The sCD83 for use according to claim 1 or claim 2, which is for use in combination with a second immunosuppressive compound wherein the first immunosuppressive compound is tacrolimus and the second immunosuppressive compound is mycophenolate mofetil.

6. The sCD83 for use according to any of claims 1 to 5, wherein CD83 and said first immunosuppressive compound are for administration within three days of each other.

7. The sCD83 for use according to any of claims 1 to 6 wherein said disease or disorder is an autoimmune disease.

8. The sCD83 for use according to any of claims 1 to 7, wherein said CD83 is administered by introducing nucleic acid encoding CD83 into the subject.

9. The sCD83 for use according to any one of claims 1 to 6, wherein said disease or disorder is rejection of transplanted tissue, and wherein the soluble CD83 is for administration to a transplant patient with said transplanted tissue and/or for administration to the transplant patient following the transplantation of the tissue.

10. The sCD83 for use according to claim 9, which is for administration with a therapeutic composition.

11. The sCD83 for use according to claim 9, wherein said therapeutic composition is an antigen associated with the transplanted tissue.

12. The sCD83 for use according to any one of claims 1 to 6, wherein said disease or disorder is rejection of transplanted tissue and wherein said sCD83 is for administration to a tissue to be transplanted after removal from a donor.

13. A combination of soluble CD83 and a first immunosuppressive compound for use to prevent, cure, or alleviate at least one symptom of a disease or disorder caused by the dysfunction or undesired function of an immune response, wherein said first immunosuppressive compound is selected from the group consisting of cyclosporine, tacrolimus, sirolimus, and anti-CD45RB mAb, wherein said disease or disorder is selected from the group consisting of an allergy; asthma; rejection of transplanted tissue; an immune response to a chronically administered substance; an autoimmune disease; and AIDS.

14. The sCD83 for use according to any one of claims 1 to 12 or the combination for use according to claim 13, wherein said each of said soluble CD83, said first immunosuppressive compound, said second immunosuppressive compound and said therapeutic composition is for simultaneous administration, or is for separate administration.

15. Use of a combination of soluble CD83 and a first immunosuppressive compound for treating a tissue to be transplanted after removal from a donor and prior to transplantation, wherein said first immunosuppressive compound is selected from the group consisting of cyclosporine, tacrolimus, sirolimus, and anti-CD45RB mAb.

16. The soluble CD83 for use according to claim 1 or the combination of soluble CD83 and a first immunosuppressive compound for use according to claim 13, wherein said autoimmune disease is selected from the group consisting of myasthenia gravis, chronic inflammatory bowel disease, ankylosing spondylitis, systemic lupus erythematosus, psoriasis, rheumatoid arthritis, and insulin-dependent diabetes mellitus.

17. The soluble CD83 for use according to claim 16 or the combination of soluble CD83 and a first immunosuppressive compound for use according to claim 16, wherein said chronic inflammatory bowel disease is Crohn's disease or ulcerative colitis.

## Patentansprüche

1. Lösliches CD83 zur Verwendung zur Vorbeugung, Heilung oder Linderung von mindestens einem Symptom einer Erkrankung oder Störung, die durch die Dysfunktion oder unerwünschte Funktion einer Immunantwort in Verbindung mit einer ersten immunsuppressiven Verbindung verursacht wurde, wobei jene erste immunsuppressive Verbindung ausgewählt ist aus Cyclosporin, Tacrolimus, Sirolimus und anti-CD45RB-monoklonaler Antikörper (mAb), wobei jene Erkrankung oder Störung ausgewählt ist aus der Gruppe, bestehend aus einer Allergie; Asthma; Abstoßung von transplantiertem Gewebe; einer Immunantwort auf eine chronisch verabreichte Substanz; einer Autoimmunerkrankung; und AIDS.

2. Das sCD83 zur Verwendung nach Anspruch 1, wobei das sCD83 und die erste immunsuppressive Verbindung für die Verabreichung in der gleichen Formulierung und auf dem gleichen Weg der Verabreichung oder zur Verabreichung in verschiedenen Formulierungen und/oder über verschiedene Verabreichungswege sind.

3. Das sCD83 zur Verwendung nach Anspruch 1 oder Anspruch 2, wobei jene erste immunsuppressive Verbindung Cyclosporin ist.

4. Das sCD83 zur Verwendung nach Anspruch 1 oder Anspruch 2, das zur Verwendung in Kombination mit einer zweiten immunsuppressiven Verbindung ist, wobei jene erste immunsuppressive Verbindung Sirolimus und jene zweite immunsuppressive Verbindung anti-CD45RB-mAb ist.

5. Das sCD83 zur Verwendung nach Anspruch 1 oder Anspruch 2, das zur Verwendung in Kombination mit einer zweiten immunsuppressiven Verbindung ist, wobei die erste immunsuppressive Verbindung Tacrolimus und die zweite immunsuppressive Verbindung Mycophenolat-Mofetil ist.

6. Das sCD83 zur Verwendung nach einem der Ansprüche 1 bis 5, wobei CD83 und jene erste immunsuppressive Verbindung zur Verabreichung innerhalb von drei Tagen voneinander sind.

7. Das sCD83 zur Verwendung nach einem der Ansprüche 1 bis 6, wobei jene Erkrankung oder Störung eine Autoimmunerkrankung ist.

8. Das sCD83 zur Verwendung nach einem der Ansprüche 1 bis 7, wobei jenes CD83 verabreicht wird durch Einführen einer Nukleinsäure, die CD83 kodiert, in das Subjekt.

9. Das sCD83 zur Verwendung nach einem der Ansprüche 1 bis 6, wobei die Erkrankung oder Störung eine Abstoßung transplantierten Gewebes ist und wobei das lösliche CD83 zur Verabreichung an einen Transplantationspatienten mit jenem transplantierten Gewebe und/oder für die Verabreichung an den Transplantationspatienten nach der Transplantation von Gewebe ist.

10. Das sCD83 zur Verwendung nach Anspruch 9, das zur Verabreichung mit einer therapeutischen Zusammensetzung ist.

11. Das sCD83 zur Verwendung nach Anspruch 9, wobei jene therapeutische Zusammensetzung ein Antigen ist das mit dem transplantierten Gewebe assoziiert ist.

12. Das sCD83 zur Verwendung nach einem der Ansprüche 1 bis 6, wobei jene Erkrankung oder Störung eine Abstoßung transplantierten Gewebes ist und wobei jenes sCD83 zur Verabreichung an ein Gewebe ist, das nach Entnahme von einem Spender zu transplantieren ist.

13. Eine Kombination von löslichem CD83 und einer ersten immunsuppressiven Verbindung zur Verwendung zur Vorbeugung, Heilung oder Linderung von mindestens einem Symptom einer Erkrankung oder Störung, die durch die Dysfunktion oder unerwünschte Funktion einer Immunantwort verursacht wurde, wobei jene erste immunsuppressive Verbindung ausgewählt ist aus der Gruppe, bestehend aus Cyclosporin, Tacrolimus, Sirolimus und anti-CD45RB-mAb, wobei jene Erkrankung oder Störung ausgewählt ist aus der Gruppe, bestehend aus einer Allergie; Asthma; Abstoßung von transplantiertem Gewebe; einer Immunantwort auf eine chronisch verabreichte Substanz; einer Autoimmunerkrankung; und AIDS.

14. Das sCD83 zur Verwendung nach einem der Ansprüche 1 bis 12 oder die Kombination zur Verwendung nach Anspruch 13, wobei jenes jedes von jenem löslichen CD83, jener ersten immunsuppressiven Verbindung, jener zweiten immunsuppressiven Verbindung und jene therapeutische Zusammensetzung zur gleichzeitigen Verabreichung oder für die getrennte Verabreichung ist.

15. Verwendung einer Kombination von löslichem CD83 und einer ersten immunsuppressiven Verbindung zur Behandlung eines Gewebes, das nach der Entfernung von einem Spender zu transplantieren ist und vor der Transplantation, wobei jene erste immunsuppressive Verbindung aus der Gruppe ausgewählt ist, bestehend aus Cyclosporin, Tacrolimus, Sirolimus und anti-CD45RB-mAb.

16. Das lösliche CD83 zur Verwendung nach Anspruch 1 oder die Kombination von löslichem CD83 und einer ersten immunsuppressiven Verbindung zur Verwendung nach Anspruch 13, wobei jene Autoimmunerkrankung ausgewählt ist aus der Gruppe, bestehend aus Myasthenia gravis, chronisch-entzündlicher Darmerkrankung, Spondylitis ankylosans, systemischem Lupus erythematodes, Schuppenflechte, rheumatoider Arthritis und Insulinabhängigem Diabetes mellitus.

17. Das lösliche CD83 zur Verwendung nach Anspruch 16 oder die Kombination von löslichen CD83 und einer ersten immunsuppressive Verbindung zur Verwendung nach Anspruch 16, wobei jene chronisch-entzündliche Darmerkrankung Morbus Crohn oder Colitis ulcerosa ist.

## Revendications

1. CD83 soluble pour une utilisation pour prévenir, guérir ou soulager au moins un symptôme d'une maladie ou d'un trouble provoqué par le dysfonctionnement ou la fonction indésirée d'une réponse immunitaire en combinaison avec un premier composé immunosuppresseur, ledit premier composé immunosuppresseur étant choisi parmi la cyclosporine, le tacrolimus, le sirolimus et un anticorps monoclonal (mAb) anti-CD45RB, ladite maladie ou ledit trouble étant choisi(e) dans le groupe consistant en une allergie ; l'asthme ; le rejet de tissu greffé ; une réponse immunitaire à une substance administrée de façon chronique ; une maladie auto-immune ; et le SIDA.

2. sCD83 pour une utilisation selon la revendication 1, dans lequel le sCD83 et le premier composé immunosuppresseur sont destinés à une administration dans la même formulation et par la même voie d'administration ou sont destinés à une administration dans différentes formulations et/ou par différentes voies d'administration.

3. sCD83 pour une utilisation selon la revendication 1 ou la revendication 2, dans lequel ledit premier composé immunosuppresseur est la cyclosporine.

4. sCD83 pour une utilisation selon la revendication 1 ou la revendication 2, qui est destiné à une utilisation en combinaison avec un second composé immunosuppresseur, ledit premier composé immunosuppresseur étant le sirolimus et ledit second composé immunosuppresseur étant un anticorps monoclonal (mAb) anti-CD45RB.

5. sCD83 pour une utilisation selon la revendication 1 ou la revendication 2, qui est destiné à une utilisation en combinaison avec un second composé immunosuppresseur, le premier composé immunosuppresseur étant le tacrolimus et le second composé immunosuppresseur étant le mycophénolate mofétil.

6. sCD83 pour une utilisation selon l'une quelconque des revendications 1 à 5, dans lequel le CD83 et ledit premier composé immunosuppresseur sont destinés à une administration dans l'espace de trois jours l'un par rapport à l'autre.

7. sCD83 pour une utilisation selon l'une quelconque des revendications 1 à 6, dans lequel ladite maladie ou ledit trouble est une maladie auto-immune.

8. sCD83 pour une utilisation selon l'une quelconque des revendications 1 à 7, dans lequel ledit CD83 est administré par introduction d'un acide nucléique codant pour CD83 dans le sujet.

9. sCD83 pour une utilisation selon l'une quelconque des revendications 1 à 6, dans lequel ladite maladie ou ledit trouble est le rejet d'un tissu greffé, et dans lequel le CD83 soluble est destiné à une administration à un patient qui a subi une greffe avec ledit tissu greffé et/ou pour une administration au patient qui a subi une greffe après la greffe du tissu.

10. sCD83 pour une utilisation selon la revendication 9, qui est destiné à une administration avec une composition thérapeutique.

11. sCD83 pour une utilisation selon la revendication 9, dans lequel ladite composition thérapeutique est un antigène associé au tissu greffé.

12. sCD83 pour une utilisation selon l'une quelconque des revendications 1 à 6, dans lequel ladite maladie ou ledit trouble est le rejet d'un tissu greffé et dans lequel ledit sCD83 est destiné à une administration à un tissu devant être greffé après retrait à partir d'un donneur.

13. Combinaison de CD83 soluble et d'un premier composé immunosuppresseur en vue d'une utilisation pour prévenir, guérir ou soulager au moins un symptôme d'une maladie ou d'un trouble provoqué par le dysfonctionnement ou la fonction indésirée d'une réponse immunitaire, ledit premier composé immunosuppresseur étant choisi dans le groupe consistant en la cyclosporine, le tacrolimus, le sirolimus et un anticorps monoclonal (mAb) anti-CD45RB, ladite maladie ou ledit trouble étant choisi(e) dans le groupe consistant en une allergie ; l'asthme ; le rejet de tissu greffé ; une réponse immunitaire à une substance administrée de façon chronique ; une maladie auto-immune ; et le SIDA.

14. sCD83 pour une utilisation selon l'une quelconque des revendications 1 à 12 ou combinaison pour une utilisation selon la revendication 13, dans lequel ou laquelle chacun dudit CD83 soluble, dudit premier composé immunosuppresseur, dudit second composé immunosuppresseur et de ladite composition thérapeutique est destiné à une administration simultanée, ou est destiné à une administration séparée.

15. Utilisation d'une combinaison de CD83 soluble et d'un premier composé immunosuppresseur pour traiter un tissu devant être greffé après retrait à partir d'un donneur et avant la greffe, dans laquelle ledit premier composé immunosuppresseur est choisi dans le groupe consistant en la cyclosporine, le tacrolimus, le sirolimus et un anticorps monoclonal (mAb) anti-CD45RB.

16. CD83 soluble pour une utilisation selon la revendication 1 ou combinaison de CD83 soluble et d'un premier composé immunosuppresseur pour une utilisation selon la revendication 13, dans lequel ladite maladie auto-immune est choisie dans le groupe consistant en la myasthénie grave, la maladie inflammatoire chronique de l'intestin, la spondylarthrite ankylosante, le lupus érythémateux disséminé, le psoriasis, la polyarthrite rhumatoïde et le diabète sucré insulino-dépendant.

17. CD83 soluble pour une utilisation selon la revendication 16 ou combinaison de CD83 soluble et d'un premier composé immunosuppresseur pour une utilisation selon la revendication 16, dans lequel ou laquelle ladite maladie inflammatoire chronique de l'intestin est la maladie de Crohn ou la colite ulcéreuse.
